# FASCICULE DE BREVET EUROPEEN

(11) **EP 1 893 237 B1**
(45) Date de publication et mention de la délivrance du brevet: **16.09.2009**
(21) Numéro de dépôt: 06778599.8
(22) Date de dépôt: 15.06.2006
(51) Int. Cl.: A61K 47/34, A61K 9/06, A61K 31/59, A61K 8/00, A61K 47/10, A61K 47/14

(54) **COMPOSITION PHARMACEUTIQUE COMPRENANT UN ELASTOMERE ORGANOPOLYSILOXANE ET UN PRINCIPE ACTIF SOLUBILISE**
PHARMAZEUTISCHE ZUSAMMENSETZUNG MIT EINEM ORGANOPOLYSILOXAN-ELASTOMER UND EINEM LÖSUNGSMITTEL-AKTIVWIRKSTOFF
PHARMACEUTICAL COMPOSITION COMPRISING AN ORGANOPOLYSILOXANE ELASTOMER AND A SOLUBILIZED ACTIVE PRINCIPLE

(30) Priorité: 17.06.2005 FR 0506183
(43) Date de publication de la demande: 05.03.2008
(73) Titulaire: Galderma Research & Development, 06410 Biot (FR)
(72) Inventeur: BARTHEZ, Nathalie, 06700 SAINT LAURENT DU VAR (FR); TONGLET, Emilie, 06250 Mougins Le Haut (FR); ORSONI, Sandrine, 06210 Mandelieu (FR); FREDON, Laurent, Chemin du Camouyer, 06330 Roquefort les Pins (FR)
(74) Mandataire: Allab, Myriam
(86) Numéro de dépôt international: PCT/FR2006/001357
(87) Numéro de publication internationale: WO 2006/134272

(56) Documents cités:
- WO-A-20/04028515
- WO-A-20/05053666
- FR-A- 2 843 962
- US-A- 6 103 250
- US-A1- 2002 111 387
- US-B1- 6 325 990

## Description

La présente invention concerne le domaine de la formulation de principes actifs en vue d'une application pharmaceutique topique.

La présente invention se rapporte plus particulièrement à une composition pharmaceutique stable, anhydre selon la revendication 1 comprenant un élastomère organopolysiloxane non adhésif et à titre de principe actif au moins un dérivé de la vitamine D de formule générale (I) sous forme solubilisée, à son procédé de préparation et à son utilisation pour le traitement topique du psoriasis et autres désordres cutanés.

La vitamine D et ses dérivés sont généralement utilisés en dermatologie dans le traitement du psoriasis car ils limitent la production excessive de cellules cutanées sur les surfaces atteintes et possèdent des avantages avérés pour le traitement de cette affection qui se caractérise notamment par la présence de lésions épaisses, squameuses et sèches.

Il est connu qu'un certain nombre de principes actifs présentant une activité thérapeutique intéressante sont sensibles à l'oxydation et subissent notamment une dégradation chimique conduisant à une perte sensible de leur activité en présence d'eau. La vitamine D ou certains des dérivés de la vitamine D sont notamment instables dans les milieux aqueux, surtout en environnement acide ; ils présentent une stabilité maximale à des valeurs de pH aux environs de 8.

En conséquence, il convient de formuler ces principes actifs dans des compositions de type anhydre.

Les compositions anhydres disponibles actuellement sur le marché, permettant la formulation de principes actifs sensibles à l'eau, tout en leur assurant une bonne stabilité chimique, sont généralement des compositions de type onguent. Ces compositions de type onguent sont constituées principalement de vaseline, et sont appelées onguents oléagineux. Or, la vaseline donne un toucher très gras et non cosmétique au produit, et laisse un résidu gras sur la peau.
Ce résidu gras empêche le patient atteint de psoriasis de pouvoir remettre ses vêtements après traitement sans risquer d'y laisser des marques grasses, ce qui n'incite pas forcément le patient à suivre son traitement. La non observance du traitement prescrit est l'une des causes principales d'échec ; l'article « Patients with psoriasis and their compliance with medication, Richardset al, J. Am. Acad Dermatol., Oct 1999, p. 581-583» indique que près de 40% des patients avec une maladie chronique telle que le psoriasis ne suivent pas leur traitement. De plus, les caractéristiques du véhicule utilisé dans les compositions pharmaceutiques sont directement liées à l'adhésion du patient à son traitement.

Les compositions de type onguent oléagineux actuellement sur le marché ne se prêtent pas toujours à la formulation du principe actif sous une forme solubilisée.

Dans les documents EP 0 255 369 et US 6 103 250, sont décrites des formulations le plus souvent à base de dérivés siliconés dans lesquelles les matières actives sensibles à l'eau sont conditionnées sous une forme dispersée. Cependant, la forme dispersée est généralement préjudiciable à une libération et/ou à une pénétration optimale de ces matières actives au niveau de la peau.

WO2005/053666 A divulgue des compositions sous forme de sprays comprenant un dérivé de la vitamine D. Il ne décrit pas les dérivés de la vitamine D correspondant à ceux de la présente invention.

FR2843962 A décrit des compositions topiques comprenant des dérivés de la vitamine D de la présente invention.

L'un des buts de la présente invention est de proposer une composition pharmaceutique anhydre destinée à une application topique et permettant de s'affranchir des inconvénients précités.

Un autre but de la présente invention est de proposer une composition pharmaceutique anhydre destinée à une application topique, présentant une stabilité prolongée, et dont le principe actif est sous une forme solubilisée.

Ainsi la présente invention a pour objet une composition pharmaceutique anhydre selon la revendication 1 destinée au traitement du psoriasis comprenant un élastomère organopolysiloxane non adhésif et, à titre de principe actif, au moins un composé dérivé de la vitamine D de formule générale (I), ledit composé étant sous une forme solubilisée dans ladite composition.

Les dérivés de vitamine D utilisés dans l'invention sont décrits dans la demande WO 03/050067 : il s'agit de composés analogues structuraux de la vitamine D qui montrent une activité sélective sur la prolifération et sur la différenciation cellulaire, et qui possèdent une activité thérapeutique vis-à-vis d'affections dermatologiques ou de troubles cutanés comme par exemple le psoriasis.

La présente invention a donc pour objet une composition pharmaceutique anhydre selon la revendication 1, caractérisée en ce qu'elle comprend un élastomère organopolysiloxane non adhésif et, à titre de principe actif, au moins un composé dérivé de la vitamine D sous forme solubilisée dans ladite composition, ledit dérivé de vitamine D répondant à la formule générale (I) suivante : 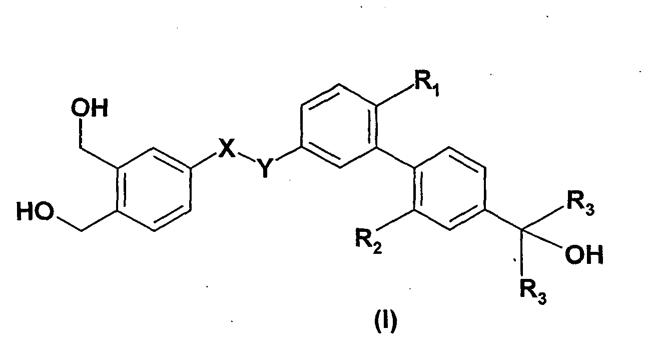 dans laquelle:
- X-Y représente une liaison choisie parmi les structures suivantes :

   -CH₂-CH₂-

   -CH₂-O-

   -O-CH₂-

   -CH₂-N(R₄)-
R₄ ayant les significations données ci-après,
   - R₁ représente un radical méthyle ou un radical éthyle,
   - R₂ représente un radical éthyle, un radical propyle ou un radical isopropyle,
   - R₃ représente un radical éthyle ou un radical trifluorométhyle,
   - R₄ représente un atome d'hydrogène, un radical méthyle, un radical éthyle ou un radical propyle.

Par forme solubilisée, on entend une dispersion à l'état moléculaire dans un liquide, aucune cristallisation de l'actif n'étant visible à l'oeil nu ni même au microscope optique en polarisation croisée.

La composition de l'invention est plus particulièrement destinée à une application topique.

La composition de l'invention est particulièrement destinée au traitement du psoriasis, qu'il soit cutané, muqueux ou unguéal, et d'autres désordres cutanés. Par autres désordres cutanés, on entend notamment des affections dermatologiques avec une composante immuno-allergique inflammatoire, avec ou sans trouble de la prolifération cellulaire, tels que le rhumatisme psoriasique ou l'atopie cutanée, telle que l'eczéma.

De préférence, la composition contient un seul composé dérivé de vitamine D de formule (I).

Le principe actif de formule (I) est à l'état solubilisé, ce qui confère aux compositions de l'invention de bonnes propriétés de libération/pénétration dans la peau du principe actif, et cela allié à une cinétique plus avantageuse. On entend par « bonne capacité de libération/pénétration » une bonne distribution de la composition de l'invention et donc du principe actif qu'elle contient, à travers le stratum corneum de la peau ainsi qu'à travers les couches sous-cutanées comme l'épiderme et le derme.

Par l'expression "composition anhydre", on entend au sens de la présente invention, une composition sensiblement exempte d'eau, c'est-à-dire présentant une teneur en eau inférieure ou égale à 5% en poids par rapport au poids total de la composition, en particulier inférieure ou égale à 3%, et notamment égale à zéro.

Selon un mode de réalisation avantageux de l'invention, la composition se présente sous la forme d'un onguent, ou d'une pommade.

De préférence, les compositions de l'invention se présentent sous la forme d'un onguent. Au sens de la présente invention et selon la pharmacopée américaine (« USP »), on entend par onguent une préparation semi-solide destinée à une application externe sur la peau ou les muqueuses. Les onguents ou pommades (de consistance plus molles que l'onguent) sont utilisés en voie topique pour de nombreuses applications, par exemple comme crèmes barrières, antiseptiques, émollients etc. Les onguents sont utilisés pour leur effet émollient, ils sont simples d'application et pénètrent facilement dans la peau.

Avantageusement les compositions selon l'invention s'avèrent, après application, dénuées d'effet collant, gras et brillant, et procurent en revanche un toucher doux. Ce nouveau type d'onguent améliore l'absorption au travers de la peau, laisse un résidu poudré non gras et apporte une facilité d'application, ce qui permet d'améliorer l'adhésion du patient à son traitement. Par ailleurs, un aspect avantageux de cette composition est l'absence d'agent conservateur.

En outre, les compositions se révèlent particulièrement efficaces pour préserver une stabilité chimique satisfaisante des principes actifs sensibles à l'oxydation, à l'eau et aux milieux aqueux d'une manière générale. Par « stabilité chimique satisfaisante », on entend une composition qui, au cours d'une période d'au moins trois mois, respectivement à température ambiante et à 40°C :
- ne présente pas de modification substantielle de son aspect macroscopique,
- comprend une teneur en principes actifs d'au moins 90 % et plus particulièrement d'au moins 95% de la teneur pondérale initiale.

Parmi les composés de formule (I) utilisables comme principe actif dans la présente invention, on peut notamment citer les suivants :
1- {5-[4'-(1-Ethyl-1-hydroxypropyl)-6-methyl-2'-propylbiphenyl-3-yloxymethyl]-2-hydroxymethylphenyl}methanol;
2- {5-[6,2'-Diethyl-4'-(1-ethyl-1-hydroxypropyl)biphenyl-3-yloxymethyl]-2-hydroxymethyl-phenyl}methanol;
3- {4-[6-Ethyl-4'-(1-ethyl-1-hydroxypropyl)-2'-propylbiphenyl-3-yloxymethyl]-2-hydroxymethylphenyl}methanol;
4- {4-[6-Ethyl-4'-(1-ethyl-1-hydroxypropyl)-2'-isopropylbiphenyl-3-yloxymethyl]-2-hydroxymethylphenyl}methanol;
5- (4-{2-[4'-(1-Ethyl-1-hydroxypropyl)-6-methyl-2'-propylbiphenyl-3-yl]ethyl}-2-hydroxymethylphenyl)methanol;
6- {4-[4'-(1-Ethyl-1-hydroxypropyl)-6-methyl-2'-propylbiphenyl-3-ylmethoxy]-2-hydroxymethylphenyl}methanol;
7- (4-{[4'-(1-Ethyl-1-hydroxypropyl)-6-methyl-2'-propylbiphenyl-3-ylamino]methyl}-2-hydroxymethylphenyl)methanol;
8- [4-({[4'-(1-Ethyl-1-hydroxypropyl)-6-methyl-2'-propylbiphenyl-3-yljmethylamino}methyl)-2-hydroxymethylphenyl]methanol;
9- [4-({Ethyl-[4'-(1-ethyl-1-hydroxypropyl)-6-methyl-2'-propylbiphenyl-3-yl]amino}methyl)-2-hydroxymethylphenyl]methanol;
10- [4-({[4'-(1-Ethyl-1-hydroxypropyl)-6-methyl-2'-propylbiphenyl-3-yl]propylamino}methyl)-2-hydroxymethylphenyl]methanol;
11- (2-Hydroxymethyl-4-{2-[6-methyl-2'-propyl-4'-(2,2,2-trifluoro-1-hydroxy-1-trifluoromethyl-ethyl)biphenyl-3-yl]ethyl}phenyl)methanol;
12- {2-Hydroxymethyl-4-[6-methyl-2'-propyl-4'-(2,2,2-trifluoro-1-hydroxy-1-trifluoromethyl-ethyl)biphenyl-3-yloxymethyl]phenyl}methanol;
13-{2-Hydroxymethyl-4-[6-methyl-2'-propyl-4'-(2,2,2-trifluoro-1-hydroxy-1-trifluoromethyl-ethyl)biphenyl-3-ylmethoxy]phenyl}methanol;
14-(2-Hydroxymethyl-4-{[6-methyl-2'-propyl-4'-(2,2,2-trifluoro-1-hydroxy-1-trifluoromethyl-ethyl)biphenyl-3-ylamino]methyl}phenyl)methanol;
15- [2-Hydroxymethyl-4-({N-methyl[6-methyl-2'-propyl-4'-(2,2,2-trifluoro-1-hydroxy-1-trifluoromethyl-ethyl)biphenyl-3-yl]amino}methyl)phenyl]methanol;
16- [4-({N-Ethyl[6-methyl-2'-propyl-4'-(2,2,2-trifluoro-1-hydroxy-1-trifluoromethylethyl)biphenyl-3-yl]amino}methyl)-2-hydroxymethylphenyl]methanol;
17- [2-Hydroxymethyl-4-({[6-methyl-2'-propyl-4'-(2,2,2-trifluoro-1-hydroxy-1-trifluoromethyl-ethyl)biphenyl-3-yl]N-propyl-amino}methyl)phenyl]methanol;
18- (4-{2-[6-Ethyl-4'-(1-ethyl-1-hydroxypropyl)-2'-propylbiphenyl-3-yl]ethyl}-2-hydroxymethylphenyl)methanol;
19- {4-[6-Ethyl-4'-(1-ethyl-1-hydroxypropyl)-2'-propylbiphenyl-3-ylmethoxy]-2-hydroxymethylphenyl}methanol;
20- (4-{[6-Ethyl-4'-(1-ethyl-1-hydroxypropyl)-2'-propylbiphenyl-3-ylamino]methyl}-2-hydroxymethylphenyl)methanol;
21- [4-({[6-Ethyl-4'-(1-ethyl-1-hydroxypropyl)-2'-propylbiphenyl-3-yl]methylamino}methyl)-2-hydroxymethylphenyl]methanol;
22- [4-({Ethyl-[6-ethyl-4'-(1-ethyl-1-hydroxypropyl)-2'-propylbiphenyl-3-yl]amino}methyl)-2-hydroxymethylphenyl]methanol;
23- [4-({[6-Ethyl-4'-(1-ethyl-1-hydroxypropyl)-2'-propylbiphenyl-3-yl]propylamino}methyl)-2-hydroxymethylphenyl]methanol;
24- (4-{2-[6-Ethyl-2'-propyl-4'-(2,2,2-trifluoro-1-hydroxy-1-trifluoromethylethyl)biphenyl-3-yl]ethyl}-2-hydroxymethylphenyl)methanol;
25- {4-[6-Ethyl-2'-propyl-4'-(2,2,2-trifluoro-1-hydroxy-1-trifluoromethylethyl)biphenyl-3-yloxymethyl]-2-hydroxymethylphenyl}methanol;
26- {4-[6-Ethyl-2'-propyl-4'-(2,2,2-trifluoro-1-hydroxy-1-trifluoromethylethyl)biphenyl-3-ylmethoxy]-2-hydroxymethylphenyl}methanol;
27- (4-{[6-Ethyl-2'-propyl-4'-(2,2,2-trifluoro-1-hydroxy-1-trifluoromethylethyl)biphenyl-3-ylamino]methyl}-2-hydroxymethylphenyl)methanol;
28- [4-({[6-Ethyl-2'-propyl-4'-(2,2,2-trifluoro-1-hydroxy-1-trifluoromethylethyl)biphenyl-3-yl]methylamino}methyl)-2-hydroxymethylphenyl]methanol;
29-[4-({N-Ethyl[6-ethyl-2'-propyl-4'-(2,2,2-trifluoro-1-hydroxy-1-trifluoromethylethyl)biphenyl-3-yl]amino}methyl)-2-hydroxymethylphenyl]methanol;
30- [4-({[6-Ethyl-2'-propyl-4'-(2,2,2-trifluoro-1-hydroxy-1-trifluoromethylethyl)biphenyl-3-yl]-N-propyl-amino}methyl)-2-hydroxymethylphenyl]methanol;
31-(4-{[4'-(1-Ethyl-1-hydroxypropyl)-6,2'-dimethylbiphenyl-3-ylamino]methyl}-2-hydroxymethylphenyl)methanol.

De façon particulièrement préférée, le dérivé de vitamine D utilisé dans la présente invention est le {4-[6-Ethyl-4'-(1-ethyl-1-hydroxy-propyl)-2'-propyl-biphenyl-3-yloxymethyl]-2-hydroxymethyl-phenyl}-méthanol (composé 3- ci-dessus), de formule (II) suivante :

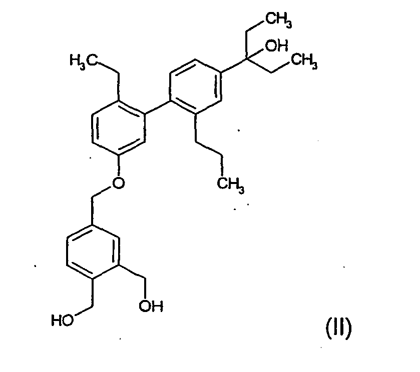

Avantageusement, la quantité de dérivé de la vitamine D sous forme solubilisée dans la composition de l'invention est de 0,00001 à 5% en poids par rapport au poids total de la composition, de préférence de 0,0001 à 3% en poids et plus particulièrement de 0,0003 à 1% en poids.

Selon un mode de réalisation avantageux de l'invention, le principe actif est solubilisé dans un solvant.
Le solvant de la présente invention est choisi parmi les composés pharmaceutiquement acceptables, c'est-à-dire les composés dont l'utilisation est en particulier compatible avec une application sur la peau, les muqueuses et/ou les fibres kératiniques. Il est généralement pâteux ou fluide, et notamment liquide, à température et pression atmosphérique ambiantes.

À titre d'agents solvants selon l'invention, on pourra notamment citer un alcool, notamment pur, tel que l'éthanol absolu, mais aussi l'oleyl macrogol 6 glycérides connu sous le nom de Labrafil M1944CS vendu par Gattefossé, le macrogol 15 hydroxystéarate vendu sous le nom de Solutol HS15 par BASF, l'huile de castor hydrogénée PEG 40 vendu sous le nom de Cremophor RH 40 par BASF, le PEG 400 vendu sous le nom de Lutrol E400 par BASF, le dimethyl isosorbide vendu sous le nom d'Arlasolve DMI par Uniqema, le PPG 15 stearyl éther vendu sous le nom d'Arlamol E par Uniqema, l'éthoxydiglycol commercialisé sous la dénomination Transcutol par GATTEFOSSE, le N-methyl-2-pyrrolidone vendu sous le nom Pharmasolve par ISP, et leurs mélanges.

De préférence, on utilise comme solvant l'éthanol absolu, ou bien un mélange d'éthanol absolu avec au moins un des composés ci-dessus, appelé alors co-solvant(s).

L'agent solvant est généralement présent dans les compositions de l'invention en une quantité d'une part suffisante pour procurer la solubilité requise du principe actif à formuler, et d'autre part compatible avec la nécessité de préserver une stabilité chimique prolongée de ce même principe actif. En d'autres termes, l'agent solvant se doit d'être inerte chimiquement vis-à-vis du principe actif.

Avantageusement, la quantité de solvant est comprise entre 1 et 25% en poids par rapport au poids total de la composition, de préférence entre 1 et 20% en poids, de préférence entre 1 et 15% en poids.
De préférence, on utilise l'éthanol absolu en quantité entre 1 et 6% en poids par rapport au poids total de la composition, de préférence entre 4 et 6% en poids, tandis que la quantité de co-solvant(s) est de 1 à 15% en poids par rapport au poids total de la composition, plus particulièrement de 1 à 10% en poids.

L'agent solvant confère également un effet bénéfique sur le taux de pénétration dans la peau des principes actifs.

Selon l'invention, la composition comprend un élastomère organopolysiloxane, et ce en quantité majoritaire en poids par rapport au poids total de la composition.

Par « élastomère organopolysiloxane », on entend tout polymère de siloxane chimiquement réticulé qui présente des propriétés viscoélastiques. Selon l'invention, un tel polymère est non réactif et non polaire.
Par ailleurs, l'élastomère selon l'invention n'a pas de propriété adhésive. Par « adhésif », on entend un matériau mou qui, placé sous forme de film mince entre deux objets, rend difficile leur séparation ; il faut exercer une certaine force pour amorcer la séparation, et fournir au total une certaine énergie.

Par propriétés viscoélastiques, on entend la capacité de l'élastomère à se déformer jusqu'à un certain point, lorsque soumis à une charge mécanique, et à reprendre sa forme originale suite au retrait de ladite charge.

Comme élastomères organopolysiloxanes utilisables dans les compositions de l'invention, on peut citer ceux qui sont notamment décrits dans les brevets US 4 980 167 et US 4 742 142. Il peut notamment s'agir de composés résultant de réactions d'addition, c'est-à-dire de produits d'hydrosilylation ou de produits de polymérisation issus de l'addition d'un organopolysiloxane ayant des groupes insaturés tels que des groupes vinyle ou allyle, en particulier liés à au moins un atome de Si-terminal avec un autre composé siliconé capable de participer à la réaction d'addition tel qu'un organohydrogénopolysiloxane.

On peut notamment citer des élastomères siliconés tels que ceux préparés par réaction de réticulation entre des polysiloxanes (A) contenant des groupes ≡Si-H tels que définis ci-dessous, avec un alpha,oméga-diène (B), en présence d'un catalyseur et d'une huile de silicone (C).

Le polysiloxane (A) contenant le motif ≡Si-H peut être représenté par les composés de formule R₃¹⁴SiO(R¹⁵₂SiO)ₐ(R¹⁶HSiO)_{b} SiR₃¹⁴ désignés ici comme le type A¹ et les composés de formule HR₂¹⁴SiO(R¹⁵₂SiO)_{c}SiR₂¹⁴H ou de formule HR₂¹⁴SiO(R¹⁵₂SiO)ₐ(R¹⁶HSiO)_{b} SiR₂¹⁴H désignés ici comme le type A². Dans ces formules, R¹⁴, R¹⁵ et R¹⁶ sont des groupes alkyles ayant de un à six atomes de carbone, a est un nombre entier variant de 0 à 250, b est un nombre entier variant de 1 à 250 et c est un nombre entier variant de 0 à 250. Le rapport molaire des composés A²: A¹ est de 0 à 20, en particulier de 0 à 5.

L'alpha, oméga-diène (B) est un composé de formule CH₂=CH(CH₂)_{d}CH=CH₂ dans laquelle d est un nombre entier variant de 1 à 20. Des exemples représentatifs de diènes alpha, oméga appropriés sont les 1,4-pentadiène, 1,5-hexadiène, 1,6-heptadiène, 1,7-octadiène, 1,8-nonadiène, 1,9-décadiène, 1,11-dodécadiène, 1,13-tétradécadiène, et 1,19-eicosadiène.

L'huile de silicone (C) volatile ou non volatile, dans laquelle est formulé l'élastomère organopolysiloxane, est un polysiloxane de faible poids moléculaire, et englobe :
- des méthylsiloxanes volatiles de faible poids moléculaire, linaires, cycliques ou ramifiés,
- des alkyl- et aryl- siloxanes de faible poids moléculaire, linéaires ou cycliques, volatiles ou non volatiles et
- des siloxanes fonctionnels de faible poids moléculaire, linéaires ou cycliques, et leurs mélanges.

Par huile de silicone « volatile », on entend toute huile de silicone susceptible de s'évaporer au contact de la peau, des muqueuses ou des fibres kératiniques en moins d'une heure, à température et pression atmosphérique ambiantes.

Avantageusement, l'huile de silicone (C) e st une huile polyorganosiloxane volatile linéaire ou cyclique, de viscosité inférieure ou égale à 6 centistokes (6.10⁻⁶ m²/s), ayant de préférence de 2 à 10 atomes de silicium, ces silicones comportant éventuellement des groupes alkyles ou alcoxy ayant de 1 à 22 atomes de carbone.

De façon plus préférée, l'huile de silicone (C) est choisie parmi les méthylsiloxanes volatiles, de faible poids moléculaire, linéaires ou cycliques.
Comme méthylsiloxanes volatiles linéaires on peut citer l'hexaméthyldisiloxane, l'octaméthyltrisiloxane, la décaméthyltétrasiloxane, la dodécaméthylpentasiloxane, la tétradécaméthylhexasiloxane, l'hexadécaméthylheptasiloxane, l'heptaméthylhexyltrisiloxane et l'heptaméthyloctyltrisiloxane.
Comme méthylsiloxanes volatiles cycliques, on peut citer le décaméthylcyclopentasiloxane, l'hexaméthylcyclotrisiloxane et le dodécaméthylcyclohexasiloxane.
Comme méthylsiloxanes volatiles ramifiées, on peut citer en particulier l'heptaméthyl-3-[(triméthylsilyl)oxy]trisiloxane, l'hexaméthyl-3,3,bis[(triméthylsilyl)oxy]trisiloxane, et la pentaméthyl [(triméthylsilyl)oxy]cyclotrisiloxane.

Conviennent également comme huiles (C) dans la présente invention des polysiloxanes de faible poids moléculaire, non volatiles, répondant à la formule générale : 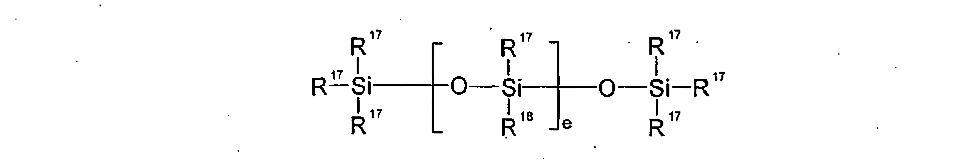 dans laquelle :
- e est tel que les polymères répondant à cette formule présentent une viscosité dans la gamme d'environ 100 à 1000 centistockes (mm²/sec), et est notamment choisi dans l'intervalle allant de 80 à 375,
- R¹⁷ et R¹⁸ sont des radicaux alkyles ayant de 1 à 20 atomes de carbone ou un groupe aryle tel qu'un groupe phényle.

Parmi ces polysiloxanes, on peut citer en particulier le polydiméthylsiloxane, le polydiéthylsiloxane, le polyméthyléthylsiloxane, le polyméthylphénylsiloxane et le polydiphénylsiloxane.

Des polysiloxanes fonctionnalisés de faible poids moléculaire peuvent être représentés par des siloxanes fluides portant des fonctions acrylamides, acrylates, amides, amino, carbinol, carboxy, chloroalkyles, époxy, glycol, cétal, mercapto, méthylester, perfluoro et silanol.

Des exemples d'élastomères ainsi obtenus par réticulation entre (A) et (B) en présence de (C) sont notamment décrits dans le brevet US 5,654,362.

De préférence, l'élastomère organopolysiloxane utilisé dans les compositions de l'invention est notamment le "ST Elastomer 10^{®}" de DOW CORNING, qui est un copolymère de diméthicone-cyclomethicone formulé dans une huile de décaméthylcyclopentasiloxane se présentant sous la forme d'un gel épais et translucide.

Ce type d'élastomère est synthétisé par réaction de réticulation similaire à celle décrite ci-dessus, à savoir préparé par réaction de réticulation entre des polysiloxanes (A) contenant des groupes ≡Si-H tels que définis ci-dessus, avec un alpha,oméga-diène (B), en présence d'un catalyseur et d'un polysiloxane linéaire ou cyclique de faible poids moléculaire (huile de silicone (C)), auxquels on ajoute des vinylsiloxanes (ou vinylsilanes) (A') contenant des groupes vinyles -CH=CH2.

En effet, il a été démontré que l'ajout de ces vinylsiloxanes (ou vinylsilanes) bloque les fonctions SiH restantes n'ayant pas réagi (« quenching agent »). Les composés (A') pouvant être utilisés pour la préparation des agents siliconés préférés selon l'invention sont tels que ceux décrits dans la demande US 5,929,164. A titre d'exemples de tels composés vinylsiloxanes ou vinylsilanes (A'), on peut citer le vinyl-t-butyldiméthylsilane, vinyldiéthylméthylsilane, vinyléthyldiméthylsilane, vinyltriéthylsilane, vinyltriméthylsilane, divinyldiméthylsilane, divinyltétraméthyldisilane, vinylpentaméthyldisiloxane, 1,3-divinyltétraméthyldisiloxane, un vinyltrisiloxane de structure (CH₃)₃SiOSi(CH=CH₂) (CH₃)OSi(CH₃)₃, 1,5-divinylhexaméthyltrisiloxane, et un oligomère divinylsiloxane ayant une structure (CH₂=CH)Me₂SiO(Me₂SiO)₈SiMe₂(CH=CH₂).

L'alpha oméga-diène(B) préféré selon l'invention est le 1,5-hexadiène.

Selon un mode de réalisation particulier, les agents siliconés selon l'invention sont préférentiellement des élastomères de polysiloxane ne contenant pas de groupement hydrophile. Par groupement hydrophile selon l'invention, on entend par exemple, un groupement de type polyoxyalkylène, ou un groupement de type glycol.

L'élastomère siliconé défini ci-dessus peut exercer notamment la fonction d'épaississant dans les compositions selon l'invention. Il peut participer en outre à leur stabilisation.

Avantageusement, la quantité d'élastomère organopolysiloxane, majoritaire dans la composition selon l'invention, peut varier substantiellement, en particulier selon la viscosité de la composition désirée.
Par quantité d'élastomère organopolysiloxane, on entend la quantité d'élastomère et d'huile de silicone dans laquelle il est formulé.

Avantageusement, la quantité d'élastomère organopolysiloxane dans une composition de l'invention est de 70 à 95 % en poids par rapport au poids total de la composition, de préférence de 80 à 95 % en poids, de préférence de 83 à 92 % en poids.

La composition peut également comprendre, en sus de l'huile de silicone (C) dans laquelle est formulé l'élastomère organopolysiloxane, au moins une huile de silicone supplémentaire. Parmi ces huiles de silicone supplémentaires, on peut citer la cyclométhicone et/ou la diméthicone présentant une viscosité comprise entre 20 et 350 centistokes. Ces huiles sont utilisées en quantité comprise entre 1 et 30% en poids, de préférence entre 1 et 15% en poids.

Selon un autre mode de réalisation avantageux, la composition comprend en outre un additif de toucher. Par additif de toucher, on entend un composé introduit dans une composition, afin d'en améliorer le toucher sur la peau.
Un tel additif est notamment choisi dans le groupe constitué par :
- l'isopropyl palmitate connu sous le nom de Crodamol IPP, l'isopropyl myristate connu sous le nom de Crodamol IPM, le C12-15 alkyl benzoate, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol, et leurs mélanges;
- les esters hydroxydés comme le lactate d'isostéaryle, le malate de di-isostéaryle, les esters de pentaérythritol, et leurs mélanges ;
- les huiles non volatiles de formule R²¹CO-OR²² dans laquelle R²¹ et R²² représentent chacun indépendamment un radical alkyle linéaire ou ramifié, un radical alcényle ou alcoxycarbonylalkyle ou alkylcarbonyloxyalkyle en C₁ à C₂₅, en particulier en C₄ à C₂₀. Des exemples de tels esters englobent l'isononanoate d'isotridécyle, le diheptanoate de PEG-4, le néopentanoate d'isostéaryle, le néopentanoate de tridécyle, l'octanoate de cétyle, le palmitate de cétyle, le ricinoléate de cétyle, le stéarate de cétyle, le myristate de cétyle, le caprate/dicaprylate de coco, l'isostéarate de décyle, l'oléate d'isodécyle, le néopentanoate d'isodécyle, le néopentanoate d'isohexyle, le palmitate d'octyle, le malate de dioctyle, l'octanoate de tridécyle, le myristate de myristyle et l'octododécanol.

L'ajout d'un additif de toucher permet d'éviter le peluchage. Préférentiellement on utilisera l'isopropyl palmitate, l'isopropyl myristate, le C12-15 alkyl benzoate ou leurs mélanges.

De préférence, la composition selon l'invention comprend :
- de 70 à 95% d'élastomère organopolysiloxane ;
- de 0 à 26% d'une huile de silicone ;
- de 0 à 15% de co-solvant;
- de 0 à 10% d'éthanol absolu ;
- de 0 à 5% d'un additif de toucher ;
- de 0,0001 à 0,5% de principe actif de formule (I).

Préférentiellement, la composition comprend :
- de 80% à 95% d'élastomère organopolysiloxane ;
- de 0 à 15% d'huile de silicone ;
- de 0 à 12% de co-solvant;
- de 0 à 6% d'éthanol absolu ;
- de 0 à 3% d'un additif de toucher ;
- de 0,0001% à 0,3% de principe actif de formule (I).

Encore plus préférentiellement, la composition comprend :
- de 83% à 92% d'élastomère organopolysiloxane ;
- de 0 à 5% d'huile de silicone ;
- de 0 à 9% de co-solvant;
- de 4 à 6% d'éthanol absolu;
- de 1 à 2% d'un additif de toucher ;
- de 0,001% à 0,3% de principe actif de formule (I).

Il est à noter que la composition selon l'invention ne comprend aucune cire ni épaississant additionnel, car cela provoque une instabilité physique des formules.

La composition selon l'invention peut cependant comprendre différents autres ingrédients. Le choix de ces ingrédients supplémentaires, de même que celui de leurs quantités respectives, est effectué de manière à ne pas porter préjudice aux propriétés attendues pour la composition. En d'autres termes, ces composés ne doivent pas affecter la stabilité chimique des principes actifs, ni leur solubilité.

Selon un mode de réalisation avantageux de l'invention, la composition comprend en outre un agent anti-oxydant choisi dans le groupe constitué par le butylhydroxytoluène (BHT), le butylhydroxyanisole (BHA), l'alpha-tocophérol DL et le propyl gallate.

La composition peut également comprendre un anti-irritant lipophile choisi dans le groupe constitué par l'acétate d'alpha-tocophérol DL, l'huile d'arbre à thé, l'extrait de thé vert et l'extrait de calendula.

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée.

La présente invention concerne encore l'utilisation d'un élastomère organopolysiloxane et à titre de principe actif au moins un dérivé de la vitamine D de formule générale (I), ledit principe actif étant sous forme solubilisée pour la préparation d'une composition pharmaceutique anhydre selon la revendication 1 destinée au traitement du psoriasis.

Dans l'utilisation ci-dessus mentionnée, la composition pharmaceutique est telle que définie ci-dessus.

La présente invention se rapporte enfin à un procédé de préparation d'une composition telle que décrite précédemment, comprenant le mélange d'au moins deux phases distinctes : une phase comprenant au moins l'élastomère organopolysiloxane, et une phase comprenant au moins le principe actif et le solvant, éventuellement le(s) co-solvant(s) dudit principe actif.
Le procédé comprend les étapes suivantes :
a) préparation de la phase A, qui comprend à l'élastomère organopolysiloxane, éventuellement mélangé avec une huile de silicone supplémentaire et/ou un additif de toucher, jusqu'à homogénéité ;
b) préparation de la phase B, par mélange d'au moins un dérivé de vitamine D de formule générale (I) avec le solvant, jusqu'à homogénéité;
c) incorporation de la phase B dans la phase A, puis homogénéisation, jusqu'à obtention d'un gel homogène.

Dé préférence, le solvant de l'étape b) est l'éthanol.
Le procédé peut éventuellement comprendre en début d'étape b) une étape de mélange des différents co-solvants dans l'éthanol, avant introduction de l'actif dérivé de vitamine D.

Selon un mode de réalisation avantageux de l'invention, le procédé est réalisé à froid, c'est-à-dire à température ambiante entre 20°C et 25°C. C'est également pour cette raison que la composition selon l'invention ne contient ni cire ni épaississant additionnel, car si de tels composés sont introduits, le procédé ne peut plus se réaliser à froid.

Les exemples ci-après illustrent l'invention, ils ne la limitent en aucune façon.

### Exemple 1 : Essais de solubilité du principe actif

La solubilité du {4-[6-Ethyl-4'-(1-ethyl-1-hydroxy-propyl)-2'-propyl-biphenyl-3-yloxymethyl]-2-hydroxymethyl-phenyl}-méthanol a été testée dans divers solvants.

| **Excipients** | **Sol max (%w/w)** |
|---|---|
| Propylene Glycol | 2.3351 |
| Ethanol 95 | > 20 |
| PEG 400 | 6.894 |
| Transcutol | >20 |
| Huile d'amande douce | 0.0932 |
| Cremophor RH40 | 3.989 |
| Arlamol E | 1.033 |
| Labrafil M1944CS | 0.936 |
| Eutanol G | 0.322 |
| Miglyol 812 | 0.3167 |
| IPP | 0.1654 |
| Mirasil CM5 | NA |
| Primol 352 | 0.0009 |

### Exemple 2 : Formulations conformes à l'invention

a) Composition 1

| **Phases** | **Nom INCI** | **% formulaire** |
|---|---|---|
| A | Cyclomethicone & dimethicone crosspolymer | 74.8 |
| A | Cyclomethicone 5 | 18.0 |
| A | Isopropyl palmitate | 1.00 |
| A | DI-alpha tocopheryl acetate | 1.00 |
| A | DI-alpha tocopherol | 0.10 |
| B | Ethanol 100 | 5.00 |
| B | {4-[6-Ethyl-4'-(1-ethyl-1-hydroxy-propyl)-2'-propyl-biphenyl-3-yloxymethyl]-2-hydroxymethyl-phenyl}-méthanol | 0.10 |

b) Composition 2

| **Phases** | **Nom INCI** | **% formulaire** |
|---|---|---|
| A | Cyclomethicone & dimethicone crosspolymer | 84.9 |
| A | Isopropyl palmitate | 1.00 |
| A | DI-alpha tocopherol | 0.10 |
| B | PEG 8 | 5.00 |
| B | Oleyl macrogol 6 glycerides | 2.9 |
| B | PEG 40 castor oil hydrogenated | 1.00 |
| B | Ethanol 100 | 5.00 |
| B | {4-[6-Ethyl-4'-(1-ethyl-1-hydroxy-propyl)-2'-propyl-biphenyl-3-yloxymethyl]-2-hydroxymethyl-phenyl}-méthanol | 0.10 |

c) Composition 3

| **Phases** | **Nom INCI** | **% formulaire** |
|---|---|---|
| A | Cyclomethicone & dimethicone crosspolymer | 91.8 |
| A | Isopropyl palmitate | 1.00 |
| A | DI-alpha tocopherol | 0.10 |
| B | Macrogol 15 hydroxystearate | 2.00 |
| B | Ethanol 100 | 5.00 |
| B | {4-[6-Ethyl-4'-(1-ethyl-1-hydroxy-propyl)-2'-propyl-biphenyl-3-yloxymethyl]-2-hydroxymethyl-phenyl}-méthanol | 0.10 |

### Mode opératoire des compositions 1,2 et 3

Fabrication à température ambiante sous lumière inactinique.

### PREPARATION DES PHASES

### Phase A

Dans le bécher formulaire homogénéiser les matières premières sous agitation rayneri (pâle défloculeuse).

### Phase B (phase active)

Homogénéiser sous agitation magnétique les différents solvants dans l'éthanol puis introduire l'actif ({4-[6-Ethyl-4'-(1-ethyl-1-hydroxy-propyl)-2'-propyl-biphenyl-3-yloxymethyl]-2-hydroxymethyl-phenyl}-méthanol) et maintenir l'agitation jusqu'à solubilisation complète de l'actif.
Incorporer la phase active au gel formé sous agitation rayneri et homogénéiser jusqu'à l'obtention d'un gel homogène.
d) Composition 4

| **Phases** | **Nom INCI** | **% formulaire** |
|---|---|---|
| A | Cyclomethicone & dimethicone crosspolymer | 84.8 |
| A | Isopropyl palmitate | 1.00 |
| B | PEG 8 | 5.00 |
| B | Oleyl macrogol 6 glycerides | 1.00 |
| B | PEG 40 castor oil hydrogéné | 3.00 |
| B | Butyl hydroxy toluène | 0.10 |
| B | Ethanol 100 | 5.00 |
| B | {4-[6-Ethyl-4'-(1-ethyl-1-hydroxy-propyl)-2'-propyl-biphenyl-3-yloxymethyl]-2-hydroxymethyl-phenyl}-méthanol | 0.10 |

e) Composition 5

| **Phases** | **Nom INCI** | **% formulaire** |
|---|---|---|
| A | Cyclomethicone & dimethicone crosspolymer | 89.8 |
| A | Isopropyl palmitate | 1.00 |
| B | PPG 15 stearyl ether | 2.00 |
| B | Dimethyl isosorbide | 2.00 |
| B | DI alpha tocopherol | 0.10 |
| B | Ethanol 100 | 5.00 |
| B | {4-[6-Ethyl-4'-(1-ethyl-1-hydroxy-propyl)-2'-propyl-biphenyl-3-yloxymethyl]-2-hydroxymethyl-phenyl}-méthanol | 0.10 |

f) Composition 6

| **Phases** | **Nom INCI** | **% formulaire** |
|---|---|---|
| A | Cyclomethicone & dimethicone crosspolymer | 83.8 |
| A | Isopropyl palmitate | 1.00 |
| B | PEG 8 | 5.00 |
| B | PEG 40 castor oil hydrogéné | 3.00 |
| B | PPG 15 stearyl ether | 2.00 |
| B | Butyl hydroxy toluene | 0.10 |
| B | Ethanol 100 | 5.00 |
| B | {4-[6-Ethyl-4'-(1-ethyl-1-hydroxy-propyl)-2'-propyl-biphenyl-3-yloxymethyl]-2-hydroxymethyl-phenyl}-méthanol | 0.10 |

### Mode opératoire des compositions 4, 5 et 6

Fabrication à température ambiante sous lumière inactinique

### PREPARATION DES PHASES

### Phase A

Dans le bécher formulaire peser le cyclomethicone dimethicone crosspolymer, l'isopropyl palmitate et le DI alpha-Tocophérol (si présent dans la composition).
Homogénéiser l'ensemble sous agitation rayneri (pâle défloculeuse).

### Phase B (phase active)

Solubiliser sous agitation magnétique le butyl hydroxy toluène (si présent dans la composition) dans l'éthanol et les différents solvants.
Introduire l'actif ({4-[6-Ethyl-4'-(1-methyl-1-hydroxy-propyl)-2'-propyl-biphenyl-3-yloxymethyl]-2-hydroxymethyl-phenyl}-méthanol) et maintenir l'agitation jusqu'à solubilisation complète de l'actif.

Incorporer la phase active au gel formé sous agitation rayneri et homogénéiser jusqu'à l'obtention d'un gel homogène.

### Exemple 3 : Etude des stabilités physique et chimique des formulations

a) Stabilité physique :
   La stabilité physique des formulations est évaluée par une observation macroscopique et microscopique de la formulation à température ambiante, 40°C et 4°C à T1 mois, T2 mois et T3 mois.
   A TA, l'observation macroscopique permet de garantir l'intégrité physique des produits. On complète la caractérisation du produit fini par une mesure du seuil d'écoulement. On utilise un rhéomètre HAAKE de type VT550 avec un mobile de mesure SVDIN.
   Les rhéogrammes sont réalisés à 25°C et à la vitesse de cisaillement de 4 s⁻¹ (γ), et en mesurant la contrainte de cisaillement. Par seuil d'écoulement (τ0 exprimé en Pascal) on entend la force nécessaire (contrainte de cisaillement minimum) pour vaincre les forces de cohésion de type Van der Waals et provoquer l'écoulement. Le seuil d'écoulement est assimilé à la valeur trouvée à la vitesse de cisaillement de 4s-1. Ces mesures sont réalisées à T24h, à T 1, T2 et T3 mois.
b) Stabilité chimique :
   Dosage de l'actif ({4-[6-Ethyl-4'-(1-ethyl-1-hydroxy-propyl)-2'-propyl-biphenyl-3-yloxymethyl]-2-hydroxymethyl-phényl}-méthanol) en HPLC à TA et 40°C à T0, T1, T2, et T3 mois.
   Résultat obtenu : R en %

### Composition 1

| **SPECIFICATIONS A T0** | | | | | | |
|---|---|---|---|---|---|---|
| Dosage | T0 : | Centrifugation | 3000 | RAS | Aspect | Gel transparent |
| analytique | 101 % | | tr/min | | macroscopique | et fluide |
| | | | 10000 tr/min | exsudat | Viscosité : T₍₄ₛ₋₁₎ en Pa.s-1 | 56 |

| | | T1 mois | T2 mois |
|---|---|---|---|
| **TA** | **Viscosité** T₍₄ₛ₋₁₎ en Pa.s-1 | 53 | 55 |
| | **Aspect macroscopique** | Conforme | Conforme |
| | **Aspect microscopique** | Conforme | Conforme |
| | **Dosage analytique** | 99.3% | 100% |
| **4°C** | **Aspect macroscopique** | Conforme | Conforme |
| | **Aspect microscopique** | Conforme | Conforme |
| **40°C** | **Aspect macroscopique** | Conforme | Conforme |
| | **Aspect microscopique** | Conforme | Conforme |
| | **Viscosité :** T₍₄ₛ₋₁₎ en Pa.s-1 | / | 48 |
| | **Dosage analytique** | 99.6% | 99.8% |

### Composition 2

| **SPECIFICATIONS A T0** | | | | | | |
|---|---|---|---|---|---|---|
| Dosage | T0: | Centrifugation | 3000 | RAS | Aspect | Gel |
| analytique | 103% | | tr/min | | macroscopique | opaque |
| | | | 10000 tr/min | Culot léger | Viscosité : T₍₄ₛ₋₁₎ en Pa.s-1 | 96 |

| | | **T1 mois** | **T2 mois** | **T3 mois** |
|---|---|---|---|---|
| **TA** | **Viscosité** : T₍₄ₛ₋₁₎ en Pa.s-1 | 108 | 91 | 103 |
| | **Aspect macroscopique** | Conforme | Conforme | Conforme |
| | **Aspect microscopique** | Conforme | Conforme | Conforme |
| | **Dosage analytique** | 101.2% | 101.5% | T25 sem : 98.8% |
| **4°C** | **Aspect macroscopique** | Conforme | Conforme | Conforme |
| | **Aspect microscopique** | Conforme | Conforme | Conforme |
| **40°C** | **Aspect macroscopique** | Conforme | Conforme | Conforme |
| | **Aspect microscopique** | Conforme | Léger jaunissement | Jaunissement |
| | **Viscosité** : T₍₄ₛ₋₁₎ en Pa.s-1 | NA | NA | 111 |
| | **Dosage analytique** | 101.1% | 104.7% | T25 sem : 98.1 % |

### Composition 3

| **SPECIFICATIONS A T0** | | | | | | |
|---|---|---|---|---|---|---|
| Dosage | T0 : | Centrifugation | 3000 | RAS | Aspect | Gel épais |
| analytique | 102.4% | | tr/min | | macroscopique | opaque |
| | | | 10000 tr/min | RAS | Viscosité : T₍₄ₛ₋₁₎ en Pa.s-1 | 236 |

| | | **T1 mois** | **T2 mois** | **T3 mois** |
|---|---|---|---|---|
| **TA** | **Viscosité :** T₍₄ₛ₋₁₎ en Pa.s-1 | 259 | 277 | 247 |
| | **Aspect macroscopique** | Conforme | Conforme | Conforme |
| | **Aspect microscopique** | Conforme | Conforme | Conforme |
| | **Dosage analytique** | 101.5% | 101.5% | 101.9% |
| **4°C** | **Aspect macroscopique** | Conforme | Conforme | Conforme |
| | **Aspect microscopique** | Conforme | Conforme | Conforme |
| **40°C** | **Aspect macroscopique** | Conforme | Conforme | Conforme |
| | **Aspect microscopique** | Conforme | Conforme | Conforme |
| | **Viscosité :** T₍₄₅₋₁₎ en Pa.s-1 | NA | NA | 431 |
| | **Dosage analytique** | 101.9% | 100.5% | 101 % |

### Composition 4

| **SPECIFICATIONS A T0** | | | | | |
|---|---|---|---|---|---|
| Dosage T0: | Centrifugation | 3000 tr/min | RAS | Aspect macroscopique | Gel opaque |
| analytique 100.6% | | 10000 tr/min | RAS | Viscosité : T₍₄ₛ₋₁₎ en Pa.s-1 | 118 |

| | | **T1 mois** | **T2 mois** | **T3 mois** |
|---|---|---|---|---|
| **TA** | **Viscosité :** T₍₄ₛ₋₁₎ en Pa.s-1 | 88 | 116 | 104 |
| | **Aspect macroscopique** | Conforme | Conforme | Conforme |
| | **Aspect microscopique** | Conforme | Conforme | Conforme |
| | **Dosage analytique** | 102.6% | 102.3% | 100.5% |
| **4°C** | **Aspect macroscopique** | Conforme | Conforme | Conforme |
| | **Aspect microscopique** | Conforme | Conforme | Conforme |
| **40°C** | **Aspect macroscopique** | Conforme | Conforme | Conforme |
| | **Aspect microscopique** | Conforme | Conforme | Conforme |
| | **Viscosité :** T₍₄ₛ₋₁₎ en Pa.s-1 | NA | NA | 122 |
| | **Dosage analytique** | 103.2% | 101.5% | 101.3% |

### Composition 5

| **SPECIFICATIONS A T0** | | | | | | |
|---|---|---|---|---|---|---|
| Dosage | T0: | Centrifugation | 3000 tr/min | NA | Aspect macroscopique | Gel épais transparent |
| analytique | 100.6% | | 10000 tr/min | NA | Viscosité : T₍₄ₛ₋₁₎ en Pa.s-1 | 185 |

### Composition 6

| **SPECIFICATIONS A T0** | | | | | | |
|---|---|---|---|---|---|---|
| Dosage | T0: | Centrifugation | 3000 tr/min | NA | Aspect macroscopique | Gel opaque |
| analytique | 101.9% | | 10000 tr/min | NA | Viscosité: T₍₄ₛ₋₁₎ en Pa.s-1 | 102 |

| | | **T1 mois** | **T2 mois** | **T3 mois** |
|---|---|---|---|---|
| **TA** | **Viscosité :** T₍₄ₛ₋₁₎ en Pa.s-1 | 107 | 106 | 108 |
| | **Aspect macroscopique** | Conforme | Conforme | Conforme |
| | **Aspect microscopique** | Conforme | Conforme | Conforme |
| | **Dosage analytique** | 102.8% | Pas de dosage | T19sem: 104.7% |
| **4°C** | **Aspect macroscopique** | Conforme | Conforme | Conforme |
| | **Aspect microscopique** | Conforme | Conforme | Conforme |
| **40°C** | **Aspect macroscopique** | Conforme | Conforme | Conforme |
| | **Aspect microscopique** | Conforme | Conforme | Conforme |
| | **Viscosité :** T₍₄ₛ₋₁₎ en Pa.s-1 | NA | NA | 115 |
| | **Dosage analytique** | 101.8% | Pas de dosage | T19sem: 103% |

### Exemple 4 : Optimisation du temps de solubilisation de la phase active

a) Réalisation de trois phases actives, deux prélèvements sont effectués sur chacune des phases après un temps d'agitation défini : 15 mn et 30 mn.

| | **Phase active Composition 3** | **Phase active Composition 4** | **Phase active Composition 5** |
|---|---|---|---|
| Macrogol 15 hydroxystearate (Solutol HS15) | 2% | | |
| Oleyl macrogol 6 glycerides | | 1% | |
| PEG 40 huile de castor hydrogénée | | 3% | |
| PEG 8 | | 5% | |
| Dimethyl isosorbide (Arlasolve DMI) | | | 2% |
| PPG 15 stearyl ether (Arlamol E) | | | 2% |
| Butyl hydroxy toluene | | 0.1% | |
| {4-[6-Ethyl-4'-(1-ethyl-1-hydroxy-propyl)-2'-propyl-biphenyl-3-yloxymethyl]-2-hydroxymethyl-phenyl}-méthanol | 0.1% | 0.1% | 0.1% |
| Ethanol | 5% | 5% | 5% |
| Mode opératoire | Solubilisation du Solutol HS15 dans l'éthanol puis solubilisation de l'actif | Homogénéisation de tous les solvants dans l'éthanol puis solubilisation de l'actif | Homogénéisation de l'Arlasolve DMI et de l'Arlamol E dans l'éthanol puis solubilisation de l'actif |
| Type d'homogénéisation | Magnétique | Magnétique | Magnétique |
| Temps d'homogénéisation des solvants avant ajout du composé A | 30 mn | 20 mn | 5 mn |
| Quantité fabriquée | Pour 1,5 kg de produit fini | | |
| Aspect macroscopique et microscopique de la phase active à TA | 15' d'agitation : 1^{er} prélèvement phase limpide, absence de cristaux RAS 20j 30' d'agitation : 2ème prélèvement RAS 20j | | |
| Aspect macroscopique et microscopique de la phase active après stockage à 4°C | 15' d'agitation : 1^{er} prélèvement RAS 20j | | |
| | 30' d'agitation : 2ème prélèvement RAS 20j | | |

Conclusion : cela met en évidence la bonne solubilisation du {4-[6-Ethyl-4'-(1-ethyl-1-hydroxy-propyl)-2'-propyl-biphenyl-3-yloxymethyl]-2-hydroxymethyl-phenyl}-méthanol au bout de 15 minutes d'agitation.
b) On fabrique ainsi la composition 3 avec optimisation du temps de solubilisation du {4-[6-Ethyl-4'-(1-ethyl-1-hydroxy-propyl)-2'-propyl-biphenyl-3-yloxymethyl]-2-hydroxymethyl-phenyl}-méthanol dans l'éthanol 100 et le macrogol 15 hydroxystearate (15 mn), et on évalue sa stabilité physique :

| **SPECIFICATIONS A T0** | | | | | | |
|---|---|---|---|---|---|---|
| Dosage analytique | Pas de dosage | Centrifugation | 3000 tr/min | NA | Aspect macroscopique | Gel épais opaque |
| | | | 10000 tr/min | NA | Viscosité T₍₄ₛ₋₁₎ en Pa.s-1 | 261 |

| | | **T1 mois** | **T2 mois** | **T3 mois** |
|---|---|---|---|---|
| **TA** | **Viscosité** T₍₄ₛ₋₁₎ en Pa.s-1 | NA | NA | 248 |
| | **Aspect macroscopique** | Conforme | Conforme | Conforme |
| | **Aspect microscopique** | Conforme | Conforme | Conforme |
| **4°C** | **Aspect macroscopique** | Conforme | Conforme | Conforme |
| | **Aspect microscopique** | Conforme | Conforme | Conforme |
| **40°C** | **Aspect macroscopique** | Conforme | Conforme | Conforme |
| | **Aspect microscopique** | Conforme | Conforme | Conforme |
| | **Viscosité** : T₍₄ₛ₋₁₎ en Pa.s-1 | NA | NA | 290 |

Conclusion : bonne solubilisation de l'actif, pas de problème de recristallisation.

### Exemple 5 : Optimisation du procédé

On fabrique des formules placébos au réacteur (IKA LR 2.ST) (quantité fabriquée 1,5 kg), et on évalue leur stabilité physique.
a) Placébo composition 3:

| **Phases** | **Nom INCI** | **% formulaire** |
|---|---|---|
| A | Cyclomethicone & dimethicone crosspolymer | 91.8 |
| A | Isopropyl palmitate | 1.00 |
| A | DI-alpha tocopherol | 0.10 |
| B | Macrogol 15 hydroxystearate | 2.00 |
| B | Ethanol 100 | 5.00 |

Mode opératoire :
Dans la cuve du réacteur introduire successivement :
b) Placébo composition 4 :

| **Phases** | **Nom INCI** | **% formulaire** |
|---|---|---|
| A | Cyclomethicone & dimethicone crosspolymer | 84.9 |
| A | Isopropyl palmitate | 1.00 |
| B | PEG 8 | 5.00 |
| B | PEG 40 castor oil hydrogéné | 3.00 |
| B | Oleyl macrogol 6 glycerides | 1.00 |
| B | Butyl hydroxy toluene | 0.10 |
| B | Ethanol 100 | 5.00 |

Mode opératoire :
Dans la cuve du réacteur introduire successivement :
- Cyclomethicone & dimethicone crosspolymer (vitesse d'agitation 7tr/mn)
- Isopropyl palmitate.
   Vitesse d'agitation 26tr/mn, vide : -0,7 bar.

Puis introduire lentement la phase solvante :
- Ethanol absolu + oleyl macrogol 6 glycerides + PEG 40 castor oil hydrogéné + PEG 8 + butyl hydroxy toluene (préalablement homogénéisés sous agitation magnétique pendant 20 mn)
Vitesse d'agitation 50 tr/mn, puis augmentation à 90 tr/mn à la fin de l'ajout de la phase solvante pour une bonne homogénéisation.

Désaération du produit sous agitation lente, vitesse 10 tr/mn, vide :-0,8 bar pendant 30 mn.

### Stabilité physique :

| | | **T0** | **T3mois** |
|---|---|---|---|
| **TA** | **Viscosité** : T₍₄ₛ₋₁₎ en Pa.s-1 | 102 | 83 |
| | **Aspect macroscopique** | Gel épais opaque | Conforme |
| **40°C** | **Aspect macroscopique** | / | Conforme |
| | **Viscosité** : T₍₄ₛ₋₁₎ en Pa.s-1 | / | 95 |

### Conclusion :

- meilleure homogénéisation des produits grâce au système d'agitation (tige à ancre) ;
- obtention de produits désaérés grâce à l'agitation sous vide.

### Exemple 6: Variation de la concentration en actif

a) Stabilité physique de la composition 3 avec variation de la concentration en {4-[6-Ethyl-4'-(1-ethyl-1-hydroxy-propyl)-2'-propyl-biphenyl-3-yloxymethyl]-2-hydroxymethyl-phenyl}-méthanol de 0,15% à 0,3% en poids par rapport au poids de la composition totale (m/m):

| | | | | | |
|---|---|---|---|---|---|
| **Concentration en {4-[6-Ethyl-4'-(1-ethyl-1-hydroxy-propyl)-2'-propyl-biphenyl-3-yloxymethyl]-2-hydroxymethyl-phenyl}-méthanol** | | **0.15%** | **0.2%** | **0.25%** | **0.3%** |
| **TA T0** | **Aspect macroscopique microscopique** Viscosité : **T**₍₄ₛ₋₁₎ en Pa.s-1 | Gel épais Opaque 241 | Gel épais Opaque 237 | Gel épais Opaque 213 | Gel épais Opaque 207 |
| **TA T3m** | **Aspect macroscopique microscopique** Viscosité : T₍₄ₛ₋₁₎ en Pa.s-1 | Conforme 252 | Conforme 256 | Conforme 238 | Conforme 225 |
| **4°C T3m** | **Aspect macroscopique microscopique** | Conforme | Conforme | Conforme | Conforme |
| **40°C T3m** | **Aspect macroscopique microscopique** Viscosité : T₍₄ₛ₋₁₎ en Pa.s-1 | Conforme 231 | Conforme 256 | Conforme 390 | Conforme 228 |

b) Stabilité physique de la composition 4 avec variation de la concentration en {4-[6-Ethyl-4'-(1-ethyl-1-hydroxy-propyl)-2'-propyl-biphenyl-3-yloxymethyl]-2-hydroxymethyl-phenyl}-méthanol de 0,15% à 0,3% (m/m):

| | | | | | |
|---|---|---|---|---|---|
| **Concentration en {4-[6-Ethyl-4'-(1-ethyl-1-hydroxy-propyl)-2'-propyl-biphenyl-3-yloxymethyl]-2-hydroxymethyl-phenyl}-méthanol** | | **0.15%** | **0.2%** | **0.25%** | **0.3%** |
| **TA T0** | **Aspect macroscopique microscopique** Viscosité : T₍₄ₛ₋₁₎ | Gel épais Opaque 98 | Gel épais Opaque 107 | Gel épais Opaque 101 | Gel épais Opaque 86 |
| **TA T3m** | **Aspect macroscopique microscopique** Viscosité : T₍₄ₛ₋₁₎ | Conforme 129 | Conforme 112 | Conforme 110 | Conforme 121 |
| **4°C T3m** | **Aspect macroscopique microscopique** | Conforme | Conforme | Conforme | Conforme |
| **40°C T3m** | **Aspect macroscopique microscopique** Viscosité : T₍₄ₛ₋₁₎ | Conforme 114 | Conforme 131 | Conforme 121 | Conforme 121 |

Les compositions sont donc stables physiquement pour des concentrations en actif entre 0,15 et 0,3% (m/m).

### Exemple 5 : ETUDE DE TOLERANCE LOCALE

Une étude de tolérance a été menée sur des placébos de formulations de référence et de la composition 2.
Traitement : application quotidienne du jour 1 au jour 6 de 20µl de la formulation sur l'oreille droite chez la souris Balb/c.
Méthode d'évaluation : Observation clinique et mesure de l'épaisseur de l'oreille de souris du jour 2 au jour 12.
Pesée des animaux le jour 1 et le jour 12.
Le placébo de la composition 2 n'est pas irritant.

### Exemple 7 : ETUDE DE TOLERANCE

Etude réalisée sur des formules placébos et composition 2 (contenant 0.1% en poids par rapport au poids total de la composition de {4-[6-Ethyl-4'-(1-ethyl-1-hydroxypropyl)-2'-propyl-biphenyl-3-yloxymethyl]-2-hydroxymethyl-phenyl}-méthanol).
Après application topique quotidienne une fois par jour pendant 6 jours de 20µl de la formulation sur l'oreille droite chez la souris Balb/c.
La composition 2 induit le même profil de réponse avec une amplitude inférieure d'environ 30% à celle de {4-[6-Ethyl-4'-(1-ethyl-1-hydroxy-propyl)-2'-propyl-biphenyl-3-yloxymethyl]-2-hydroxymethyl-phenyl}-méthanol à 0.1% dans l'éthanol ; elle n'induit pas d'effet hypercalcémiant et aucune perte de poids
Le placébo de cette même formule n'induit pas de réponse inflammatoire.

### Exemple 8 : ETUDE DE LIBERATION/PENETRATION

But: comparer l'absorption percutanée in vitro du {4-[6-Ethyl-4'-(1-ethyl-1-hydroxypropyl)-2'-propyl-biphenyl-3-yloxymethyl]-2-hydroxymethyl-phenyl}-méthanol radiomarqué à travers la peau humaine à 0.1 % (m/m) entre la composition 2 et le {4-[6-Ethyl-4'-(1-ethyl-1-hydroxy-propyl)-2'-propyl-biphenyl-3-yloxymethyl]-2 hydroxymethyl-phenyl}-méthanol formulé dans un véhicule connu pour sa grande tolérance (témoin).
Alors que la formule témoin est prise pour référence et donne 100% d'absorption de {4-[6-Ethyl-4'-(1-ethyl-1-hydroxy-propyl)-2'-propyl-biphenyl-3-yloxymethyl]-2-hydroxymethyl-phenyl}-méthanol, la composition 2 donne 224% d'absorption de {4-[6-Ethyl-4'-(1-ethyl-1-hydroxy-propyl)-2'-propyl-biphenyl-3-yloxymethyl]-2-hydroxymethylphenyl}-méthanol.

Les compositions selon l'invention permettent donc une pénétration deux fois plus importante de l'actif.

## Revendications

1. Composition pharmaceutique anhydre, **caractérisée en ce qu'**elle comprend un élastomère organopolysiloxane et, à titre de principe actif, au moins un dérivé de la vitamine D, sous forme solubilisée, ledit dérivé de la vitamine D répondant à la formule générale (I) suivante: 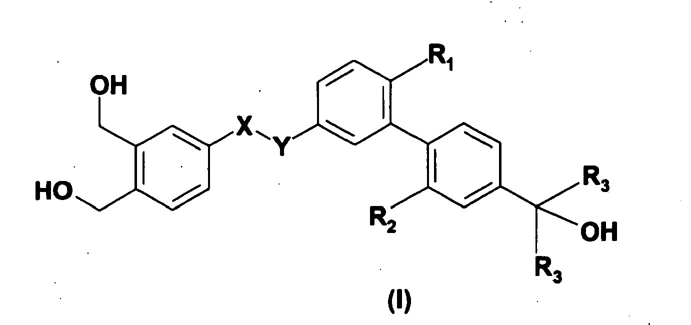 dans laquelle :
- X-Y représente une liaison choisie parmi les structures suivantes :
-CH₂-CH₂-
-CH2-O-
-O-CH₂-
-CH₂-N(R₄)-
R₄ ayant les significations données ci-après,
- R₁ représente un radical méthyle ou un radical éthyle,
- R₂ représente un radical éthyle, un radical propyle ou un radical isopropyle,
- R₃ représente un radical éthyle ou un radical trifluorométhyle,
- R₄ représente un atome d'hydrogène, un radical méthyle, un radical éthyle ou un radical propyle,
ledit élastomère organopolysiloxane, n'ayant pas de propriété adhésive, est formulé dans au moins une huile de silicone volatile choisie parmi les huiles polyorganosiloxanes linéaires ou cycliques ayant de 2 à 10 atomes de silicium, et comportant éventuellement des groupes alkyles ou alcoxy de 1 à 22 atomes de carbone,
la quantité dudit élastomère organopolysiloxane est de 70 à 95% en poids par rapport au poids total de la composition, de préférence de 80 à 95% en poids, et
ladite composition étant sous la forme d'un onguent ou d'une pommade.

2. Composition selon la revendication 1, **caractérisée en ce que** le dérivé de la vitamine D est choisi dans le groupe constitué par :
1- {5-[4'-(1-Ethyl-1-hydroxypropyl)-6-methyl-2'-propylbiphenyl-3-yloxymethyl]-2-hydroxymethylphenyl}methanol;
2- {5-[6,2'-Diethyl-4'-(1-ethyl-1-hydroxypropyl)biphenyl-3-yloxymethyl]-2-hydroxymethyl-phenyl}methanol;
3- {4-[6-Ethy)-4'-(1-ethy)-1-hydroxypropyl}-2'-propylbiphenyl-3-yloxymethyl]-2-hydroxymethylphenyl}methanol;
4- {4-[6-Ethyl-4'-(1-ethyl-1-hydroxypropyl)-2'-isopropylbiphenyl-3-yloxymethyl]-2-hydroxymethylphenyl}methanol;
5- (4-{2-[4'-(1-Ethyl-1-hydroxypropyl)-6-methyl-2'-propylbiphenyl-3-yl]ethyl}-2-hydroxymethylphenyl)methanol;
6- {4-[4'-(1-Ethyl-1-hydroxypropyl)-6-methyl-2'-propylbiphenyl-3-ylmethoxy]-2-hydroxymethylphenyl}methanol;
7- (4-{[4'-(1-Ethyl-1-hydroxypropyl)-6-methyl-2'-propylbiphenyl-3-ylamino]methyl}-2-hydroxymethylphenyl)methanol;
8- [4-({[4'-(1-Ethyl-1-hydroxypropyl)-6-methyl-2'-propylbiphenyl-3-yl]methylamino}methyl)-2-hydroxymethylphenyl]methanol;
9- [4-({Ethyl-[4'-(1-ethyl-1-hydroxypropyl)-6-methyl-2'-propylbiphenyl-3-yl]amino}methyl)-2-hydroxymethylphenyl]methanol;
10- [4-({[4'-(1-Ethyl-1-hydroxypropyl)-6-methyl-2'-propylbiphenyl-3-yl]propylamino}methyl)-2-hydroxymethylphenyl]methanol;
11-(2-Hydroxymethyl-4-{2-[6-methyl-2'-propyl-4'-(2,2,2-trifluoro-1-hydroxy-1-trifluoromethyl-ethyl)biphenyl-3-yl]ethyl}phenyl)methanol;
12-{2-Hydroxymethyl-4-[6-methyl-2'-propyl-4'-(2,2,2-trifluoro-1-hydroxy-1-trifluoromethyl-ethyl)biphenyl-3-yloxymethyl]phenyl}methanol;
13- {2-Hydroxymethyl-4-[6-methyl-2'-propyl-4'-(2,2,2-trifluoro-1-hydroxy-1-trifluoromethyl-ethyl)biphenyl-3-ylmethoxy]phenyl}methanol;
14-(2-Hydroxymethyl-4-{[6-methyl-2'-propyl-4'-(2,2,2-trifluoro-1-hydroxy-1-trifluoromethyl-ethyl)biphenyl-3-ylamino]methyl}phenyl)methanol;
15- [2-Hydroxymethyl-4-({N-methyl[6-methyl-2'-propyl-4'-(2,2,2-trifluoro-1-hydroxy-1-trifluoromethyl-ethyl)biphenyl-3-yl]amino}methyl)phenyl]methanol;
16- [4-({N-Ethyl[6-methyl-2'-propyl-4'-(2,2,2-trifluoro-1-hydroxy-1-trifluoromethyl-ethyl)biphenyl-3-yl]amino}methyl)-2-hydroxymethylphenyl]methanol;
17- [2-Hydroxymethyl-4-({[6-methyl-2'-propyl-4'- (2,2,2-trifluoro-1-hydroxy-1-trifluoromethyl-ethyl)biphenyl-3-yl]N-propyl-amino}methyl)phenyl]methanol;
18- (4-{2-[6-Ethyl-4'-(1-ethyl-1-hydroxypropyl)-2'-propylbiphenyl-3-yl]ethyl}-2-hydroxymethylphenyl)methanol;
19- {4-[6-Ethyl-4'-(1-ethyl-1-hydroxypropyl)-2'-propylbiphenyl-3-ylmethoxy]-2-hydroxymethylphenyl}methanol;
20- (4-{[6-Ethyl-4'-(1-ethyl-1-hydroxypropyl)-2'-propylbiphenyl-3-ylamino]methyl}-2-hydroxymethylphenyl)methanol;
21- [4-({[6-Ethyl-4'-(1-ethyl-1-hydroxypropyl)-2'-propylbiphenyl-3-yl]methylamino}methyl)-2-hydroxymethylphenyl]methanol;
22- [4-({Ethyl-[6-ethyl-4'-(1-ethyl-1-hydroxypropyl)-2'-propylbiphenyl-3-yl]amino}methyl)-2-hydroxymethylphenyl]methanol;
23- [4-({[6-Ethyl-4'-(1-ethyl-1-hydroxypropyl)-2'-propylbiphenyl-3-yl]propylamino}methyl)-2-hydroxymethylphenyl]methanol;
24- (4-{2-[6-Ethyl-2'-propyl-4-(2,2,2-trifluoro-1-hydroxy-1-trifluoromethyl-ethyl)biphenyl-3-yl]ethyl}-2-hydroxymethylphenyl)methanol;
25- {4-[6-Ethyl-2'-propyl-4'-(2,2,2-trifluoro-1-hydroxy-1-trifluoromethyl-ethyl)biphenyl-3-yloxymethyl]-2-hydroxymethylphenyl}methanol;
26- {4-[6-Ethyl-2'-propyl-4'-(2,2,2-trifluoro-1-hydroxy-1-trifluoromethyl-ethyl)biphenyl-3-ylmethoxy]-2-hydroxymethylphenyl}methanol;
27- (4-{[6-Ethyl-2'-propyl-4'-(2,2,2-trifluoro-1-hydroxy-1-trifluoromethyl-ethyl)biphenyl-3-ylamino]methyl}-2-hydroxymethylphenyl)methanol;
28- [4-({[6-Ethyl-2'-propyl-4'-(2,2,2-trifluoro-1-hydroxy-1-trifluoromethyl-ethyl)biphenyl-3-yl]methylamino}methyl)-2-hydroxymethylphenyl]methanol;
29- [4-({N-Ethyl[6-ethyl-2'-propyl-4'-(2,2,2-trifluoro-1-hydroxy-1-trifluoromethyl-ethyl)biphenyl-3-yl]amino}methyl)-2-hydroxymethylphenyl]methanol;
30- [4-({[6-Ethyl-2'-propyl-4'-(2,2,2-trifluoro-1-hydroxy-1-trifluoromethyl-ethyl)biphenyl-3-yl]-N-propyl-amino}methyl)-2-hydroxymethylphenyl]methanol;
31-(4-{[4'-(1-Ethyl-1-hydroxypropyl)-6,2'-dimethylbiphenyl-3-ylamino]methyl}-2-hydroxymethylphenyl)methanol.

3. Composition selon la revendication 1 ou 2, **caractérisée en ce que** le dérivé de la vitamine D est le {4-[6-Ethyl-4'-(1-ethyl-1-hydroxy-propyl)-2'-propyl-biphenyl-3-yloxymethyl]-2-hydroxymethyl-phenyl}-méthanol.

4. Composition selon l'une quelconque des revendications 1 à 3, **caractérisée en ce qu'**elle est destinée à une application topique.

5. Composition selon l'une quelconque des revendications 1 à 4, **caractérisée en ce** ledit principe actif est solubilisé dans un solvant.

6. Composition selon la revendication 5, **caractérisée en ce que** le solvant est choisi dans le groupe constitué par l'éthanol absolu, l'oléyl macrogol 6 glycérides, le macrogol 15 hydroxystéarate, l'huile de castor hydrogénée PEG 40, le PEG 400, le dimethyl isosorbide, le PPG 15 stearyl ether, l'éthoxydiglycol, le N-methyl-2-pyrrolidone, et leurs mélanges.

7. Composition selon la revendication 5 ou 6, **caractérisée en ce que** le solvant est un mélange d'éthanol absolu avec au moins un co-solvant choisi parmi l'oléyl macrogol 6 glycérides, le macrogol 15 hydroxystéarate, l'huile de castor hydrogénée PEG 40, le PEG 400, le diméthyl isosorbide, le PPG 15 stearyl ether, l'éthoxydiglycol et le N-methyl-2-pyrrolidone.

8. Composition selon l'une quelconque des revendications 1 à 7, **caractérisée en ce qu'**elle comprend un additif de toucher choisi dans le groupe constitué par l'isopropyl palmitate, l'isopropyl myristate, le C12-15 alkyl benzoate et leurs mélanges.

9. Composition selon l'une quelconque des revendications 1 à 8, **caractérisée en ce que** la quantité de dérivé de la vitamine D sous forme solubilisée est de 0,00001 à 5% en poids par rapport au poids total de la composition.

10. Composition selon l'une quelconque des revendications 1 à 9, **caractérisée en ce que** la quantité de dérivé de la vitamine D sous forme solubilisée est de 0,0001 à 3% en poids.

11. Composition selon l'une quelconque des revendications 1 à 10, **caractérisée en ce que** la quantité de dérivé de la vitamine D sous forme solubilisée est de 0,0003 à 1% en poids.

12. Composition selon l'une quelconque des revendications 5 à 11, **caractérisée en ce que** la quantité de solvant est de 1 à 25% en poids par rapport au poids total de la composition, de préférence de 1 à 20% en poids et plus particulièrement de 1 à 15% en poids.

13. Composition selon l'une quelconque des revendications 1 à 12, **caractérisée en ce qu'**elle comprend en outre un agent anti-oxydant choisi dans le groupe constitué par le butylhydroxytoluène (BHT), le butylhydroxyanisole (BHA), l'alpha-tocophérol DL, le propyl galate.

14. Composition selon l'une quelconque des revendications 1 à 13, **caractérisée en ce qu'**elle comprend un anti-irritant lipophile choisi dans le groupe constitué par l'acétate d'alpha-tocophérol DL, l'huile d'arbre à thé, l'extrait de thé vert et l'extrait de calendula.

15. Utilisation d'un élastomère organopolysiloxane et a titre de principe actif au moins un dérivé de la vitamine D de formule générale (I), ledit principe actif étant sous une forme solubilisée pour la préparation d'une composition pharmaceutique anhydre selon la revendication 1 destinée au traitement du psoriasis,

16. Utilisation selon la revendication 15, dans laquelle la composition est telle que définie à l'une quelconque des revendications 2 à 14.

17. Procédé de préparation d'une composition selon l'une des revendications 1 à 14, **caractérisé en ce qu'**il comprend les étapes suivantes :
a) préparation de la phase A, qui comprend l'élastomère organopolysiloxane, éventuellement mélangé avec une huile de silicone supplémentaire et/ou un additif de toucher, jusqu'à homogénéité;
b) préparation de la phase B, par mélange d'au moins un dérivé de vitamine D de formule générale (I) avec le solvant, jusqu' homogénéisation;
c) incorporation de la phase B dans la phase A, puis homogénéisation, jusqu'à obtention d'un gel homogène.

18. Procédé selon la revendication 17, **caractérisé en ce que** le solvant est l'éthanol.

19. Procédé selon la revendication 18, **caractérisé en ce qu'**il comprend en début d'étape b) une étape de mélange des différents co-solvants dans l'éthanol, avant introduction de l'actif dérivé de vitamine D.

20. Procédé selon l'une des revendications 17 à 19, **caractérisé en ce qu'**il est réalisé à froid.

## Claims

1. Anhydrous pharmaceutical composition, **characterized in that** it comprises an organopolysiloxane elastomer and, as active ingredient, at least one vitamin D derivative, in a solubilized form, said vitamin D derivative corresponding to general formula (I) below: 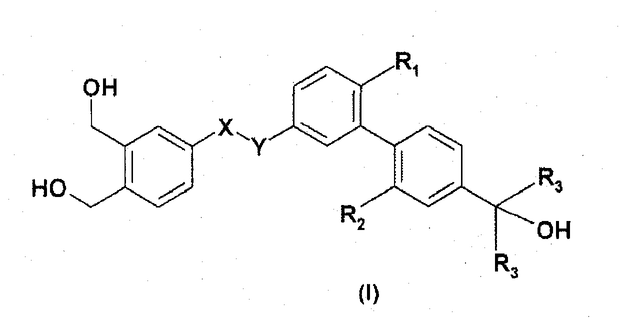 in which:
X-Y represents a bond chosen from the following structures:
-CH₂-CH₂-
--CH₂-O-
-O-CH₂-
-CH₂-N(R₄) -
R₄ having the meanings given hereinafter,
- R₁ represents a methyl radical or an ethyl radical,
- R₂ represents an ethyl radical, a propyl radical or an isopropyl radical,
- R₃ represents an ethyl radical or a trifluoromethyl radical,
- R₄ represents a hydrogen atom, a methyl radical, an ethyl radical or a propyl radical,
said organopolysiloxane elastomer, which does not have an adhesive property, is formulated in at least one volatile silicone oil chosen from linear or cyclic polyorganosiloxane oils containing from 2 to 10 silicon atoms, and optionally comprising alkyl or alkoxy groups containing from 1 to 22 carbon atoms,
the amount of said organopolysiloxane elastomer is from 70% to 95% by weight relative to the total weight of the composition, preferably from 80% to 95% by weight, and
said composition being in the form of a salve or of an ointment.

2. Composition according to Claim 1, **characterized in that** the vitamin D derivative is chosen from the group consisting of:
1- {5-[4'-(1-ethyl-1-hydroxypropyl)-6-methyl-2'-propylbiphenyl-3-yloxymethyl]-2-hydroxymethylphenyl}methanol;
2- {5-[6,2'-diethyl-4'-(1-ethyl-1-hydroxypropyl)biphenyl-3-yloxymethyl]-2-hydroxymethylphenyl}methanol;
3- {4-[6-ethyl-4'-(1-ethyl-1-hydroxypropyl)-2'-propylbiphenyl-3-yloxymethyl]-2-hydroxymethylphenyl}methanol;
4- {4-[6-ethyl-4'-(1-ethyl-1-hydroxypropyl)-2'-isopropylbiphenyl-3-yloxymethyl]-2-hydroxymethylphenyl}methanol;
5- (4-{2-[4'-(1-ethyl-1-hydroxypropyl)-6-methyl-2'-propylbiphenyl-3-yl]ethyl}-2-hydroxymethylphenyl)methanol;
6- {4-[4'-(1-ethyl-1-hydroxypropyl)-6-methyl-2'-propylbiphenyl-3-ylmethoxy]-2-hydroxymethylphenyl}methanol;
7- (4-{[4'-(1-ethyl-1-hydroxypropyl)-6-methyl-2'-propylbiphenyl-3-ylamino]methyl}-2-hydroxymethylphenyl)methanol;
8- [4-({[4'-(1-ethyl-1-hydroxypropyl)-6-methyl-2'-propylbiphenyl-3-yl]methylamino}methyl)-2-hydroxymethylphenyl]methanol;
9- [4-({ethyl-[4'-(1-ethyl-1-hydroxypropyl)-6-methyl-2'-propylbiphenyl-3-yl]amino}methyl)-2-hydroxymethylphenyl]methanol;
10- [4-({[4'-(1-ethyl-1-hydroxypropyl)-6-methyl-2'-propylbiphenyl-3-yl]propylamino}methyl)-2-hydroxymethylphenyl]methanol;
11- (2-hydroxymethyl-4-{2-[6-methyl-2'-propyl-4'-(2;2,2-trifluoro-1-hydroxy-1-trifluoromethylethyl)biphenyl-3-yl]ethyl}phenyl)methanol;
12- {2-hydroxymethyl-4-[6-methyl-2'-propyl-4'-(2,2,2-trifluoro-1-hydroxy-1-trifluoromethylethyl)biphenyl-3-yloxymethyl]phenyl}methanol;
13- {2-hydroxymethyl-4-[6-methyl-2'-propyl-4'-(2,2,2-trifluoro-1-hydroxy-1-trifluoromethylethyl)biphenyl-3-ylmethoxy]phenyl}methanol;
14- (2-hydroxymethyl-4-{[6-methyl-2'-propyl-4'-(2,2,2-trifluoro-1-hydroxy-1-trifluoromethylethyl)biphenyl-3-ylamino]methyl}phenyl)-methanol;
15- [2-hydroxymethyl-4-({N-methyl-[6-methyl-2'-propyl-4'-(2,2,2-trifluoro-1-hydroxy-1-trifluoromethylethyl)biphenyl-3-yl]amino}-methyl)phenyl]methanol;
16- [4-({N-ethyl-[6-methyl-2'-propyl-4'-(2,2,2-trifluoro-1-hydroxy-1-trifluoromethylethyl)-biphenyl-3-yl]amino}methyl)-2-hydroxymethylphenyl]methanol;
17- [2-hydroxymethyl-4-({[6-methyl-2'-propyl-4'-(2,2,2-trifluoro-1-hydroxy-1-trifluoromethylethyl)biphenyl-3-yl]N-propylamino}methyl)-phenyl]methanol;
18- (4-{2-[6-ethyl-4'-(1-ethyl-1-hydroxypropyl)-2'-propylbiphenyl-3-yl]ethyl}-2-hydroxymethylphenyl)methanol;
19- {4-[6-ethyl-4'-(1-ethyl-1-hydroxypropyl)-2'-propylbiphenyl-3-ylmethoxy]-2-hydroxymethylphenyl}methanol;
20- (4-{[6-ethyl-4'-(1-ethyl-1-hydroxypropyl)-2'-propylbiphenyl-3-ylamino]methyl}-2-hydroxymethylphenyl)methanol;
21- [4-({[6-ethyl-4'-(1-ethyl-1-hydroxypropyl)-2'-propylbiphenyl-3-yl]methylamino}methyl)-2-hydroxymethylphenyl]methanol;
22- [4-({ethyl-[6-ethyl-4'-(1-ethyl-1-hydroxypropyl)-2'-propylbiphenyl-3-yl]amino}methyl)-2-hydroxymethylphenyl]methanol;
23- [4-({[6-ethyl-4'-(1-ethyl-1-hydroxypropyl)-2'-propylbiphenyl-3-yl]propylamino}methyl)-2-hydroxymethylphenyl]methanol;
24- (4-{2-[6-ethyl-2'-propyl-4'-(2,2,2-trifluoro-1-hydroxy-1-trifluoromethylethyl)biphenyl-3-yl]ethyl}-2-hydroxymethylphenyl)methanol;
25- {4-[6-ethyl-2'-propyl-4'-(2,2,2-trifluoro-1-hydroxy-1-trifluoromethylethyl)biphenyl-3-yloxymethyl]-2-hydroxymethylphenyl}methanol;
26- {4-[6-ethyl-2'-propyl-4'-(2,2,2-trifluoro-1-hydroxy-1-trifluoromethylethyl)biphenyl-3-ylmethoxy]-2-hydroxymethylphenyl}methanol;
27- (4-{[6-ethyl-2'-propyl-4'-(2,2,2-trifluoro-1-hydroxy-1-trifluoromethylethyl)biphenyl-3-yl-amino]methyl}-2-hydroxymethylphenyl)methanol;
28- [4-({[6-ethyl-2'-propyl-4'-(2,2,2-trifluoro-1-hydroxy-1-trifluoromethylethyl)biphenyl-3-yl]methylamino}methyl)-2-hydroxymethylphenyl]methanol;
29- [4-({N-ethyl[6-ethyl-2'-propyl-4'-(2,2,2-trifluoro-1-hydroxy-1-trifluoromethylethyl)-biphenyl-3-yl]amino}methyl)-2-hydroxymethylphenyl]methanol;
30- [4-({[6-ethyl-2'-propyl-4'-(2,2,2-trifluoro-1-hydroxy-1-trifluoromethylethyl)biphenyl-3-yl]-N-propylamino}methyl)-2-hydroxymethylphenyl]methanol;
31- (4-{[4'-(1-ethyl-1-hydroxypropyl)-6,2'-dimethylbiphenyl-3-ylamino]methyl}-2-hydroxymethylphenyl)methanol.

3. Composition according to Claim 1 or 2, **characterized in that** the vitamin D derivative is {4-[6-ethyl-4'-(1-ethyl-1-hydroxypropyl)-2'-propylbiphenyl-3-yloxymethyl]-2-hydroxymethylphenyl}-methanol.

4. Composition according to any one of Claims 1 to 3, **characterized in that** it is for topical application.

5. Composition according to any one of Claims 1 to 4, **characterized in that** said active ingredient is solubilized in a solvent.

6. Composition according to Claim 5, **characterized in that** the solvent is chosen from the group consisting of absolute ethanol, oleyl macrogol 6 glycerides, macrogol 15 hydroxystearate, PEG 40 hydrogenated castor oil, PEG 400, dimethyl isosorbide, PPG 15 stearyl ether, ethoxydiglycol, N-methyl-2-pyrrolidone, and mixtures thereof.

7. Composition according to Claim 5 or 6, **characterized in that** the solvent is a mixture of absolute ethanol with at least one cosolvent chosen from oleyl macrogol 6 glycerides, macrogol 15 hydroxystearate, PEG 40 hydrogenated castor oil, PEG 400, dimethyl isosorbide, PPG 15 stearyl ether, ethoxydiglycol and N-methyl-2-pyrrolidone.

8. Composition according to any one of Claims 1 to 7, **characterized in that** it comprises a feel additive chosen from the group consisting of isopropyl palmitate, isopropyl myristate, C₁₂-C₁₅ alkyl benzoate, and mixtures thereof.

9. Composition according to any one of Claims 1 to 8, **characterized in that** the amount of vitamin D derivative in a solubilized form is from 0.00001% to 5% by weight relative to the total weight of the composition.

10. Composition according to any one of Claims 1 to 9, **characterized in that** the amount of vitamin D derivative in a solubilized form is from 0.0001% to 3% by weight.

11. Composition according to any one of Claims 1 to 10, **characterized in that** the amount of vitamin D derivative in a solubilized form is from 0.0003% to 1% by weight.

12. Composition according to any one of Claims 5 to 11, **characterized in that** the amount of solvent is from 1% to 25% by weight relative to the total weight of the composition, preferably from 1% to 20% by weight, and more particularly from 1% to 15% by weight.

13. Composition according to any one of Claims 1 to 12, **characterized in that** it also comprises an anti-oxidizing agent chosen from the group consisting of butylhydroxytoluene (BHT), butylhydroxyanisole (BHA), DL-alpha-tocopherol and propyl gallate.

14. Composition according to any one of Claims 1 to 13, **characterized in that** it comprises a lipophilic anti-irritant chosen from the group consisting of DL-alpha-tocopherol acetate, tea tree oil, green tea extract and calendula extract.

15. Use of an organopolysiloxane elastomer and, as active ingredient, at least one vitamin D derivative of general formula (I), said active ingredient being in a solubilized form, for the preparation of an anhydrous pharmaceutical composition according to Claim 1 for use in the treatment of psoriasis.

16. Use according to Claim 15, in which the composition is as defined in any one of Claims 2 to 14.

17. Process for preparing a composition according to one of Claims 1 to 14, **characterized in that** it comprises the following steps:
a) preparing phase A, which comprises the organopolysiloxane elastomer, optionally mixed with an additional silicone oil and/or a feel additive, to homogeneity;
b) preparing phase B, by mixing at least one vitamin D derivative of general formula (I) with the solvent, to homogeneity;
c) incorporating phase B into phase A, and then homogenizing until a homogeneous gel is obtained.

18. Process according to Claim 17, **characterized in that** the solvent is ethanol.

19. Process according to Claim 18, **characterized in that** it comprises, at the beginning of step b), a step of mixing the various cosolvents in ethanol, before introducing the vitamin D-derived active agent.

20. Process according to one of Claims 17 to 19, **characterized in that** it is carried out under cold conditions.

## Patentansprüche

1. Wasserfreie pharmazeutische Zusammensetzung, **dadurch gekennzeichnet, dass** sie ein elastomeres Organopolysiloxan und als Wirkstoff mindestens ein Vitamin D-Derivat in solubilisierter Form enthält, wobei das Vitamin D-Derivat der folgenden allgemeinen Formel entspricht: 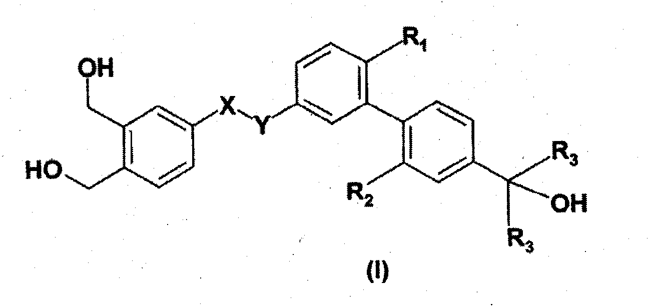 in der Formel:
X-Y bedeutet eine Bindung, die unter den folgenden Strukturen ausgewählt ist:
- CH₂-CH₂-
- CH₂-O-
- O-CH--
- CH₂-N(R₄)-
wobei R₄ die unten angegebenen Bedeutungen aufweist,
R₁ bedeutet eine Methylgruppe oder eine Ethylgruppe,
R₂ bedeutet eine Ethylgruppe, eine Propylgruppe oder eine Isopropylgruppe,
R₃ bedeutet eine Ethylgruppe oder eine Trifluormethylgruppe,
R₄ bedeutet ein Wasserstoffatom, eine Methylgruppe, eine Ethylgruppe oder eine Propylgruppe,
wobei das elastomere Organopolysiloxan, das keine adhäsiven Eigenschaften besitzt, in mindestens einem flüchtigen Siliconöl formuliert ist, das unter den linearen oder cyclischen Polyorganosiloxanölen mit 2 bis 10 Kohlenstoffatomen ausgewählt ist, die gegebenenfalls Alkyl- oder Alkoxygruppen mit 1 bis 22 Kohlenstoffatomen aufweisen,
wobei der Mengenanteil des elastomeren Organopolysiloxans im Bereich von 70 bis 95 Gew.-%, bezogen auf das Gesamtgewicht der Zusammensetzung, und vorzugsweise im Bereich von 80 bis 95 Gew.-% liegt, und
wobei die Zusammensetzung in Form einer Salbe oder Pomade vorliegt.

2. Zusammensetzung nach Anspruch 1, **dadurch gekennzeichnet, dass** das Vitamin D-Derivat ausgewählt ist unter:
1 -{5-[4'-(1-Ethyl-1-hydroxypropyl)-6-methyl-2'-propylbiphenyl-3-yloxymethyl]-2-hydroxymethylphenyl}methanol;
2- {5-[6,2'-Diethyl-4'-(1-ethyl-1-hydroxypropyl)biphenyl-3-yloxymethyl]-2-hydroxymethyl-phenyl}methanol;
3- {4-[6-Ethyl-4'-(1-ethyl-1-hydroxypropyl)-2'-propylbiphenyl-3-yloxymethyl]-2-hydroxymethylphenyl}methanol;
4- {4-[6-Ethyl-4'-(1-ethyl-1-hydroxypropyl)-2'-isopropylbiphenyl-3-yloxymethyl]-2-hydroxymethylphenyl}methanol;
5- (4-{2-[4'-(1-Ethyl-1-hydroxypropyl)-6-methyl-2'-propylbiphenyl-3-yl]ethyl}-2-hydroxymethylphenyl)methanol;
6- {4-[4'-(1-Ethyl-1-hydroxypropyl)-6-methyl-2'-propylbiphenyl-3-ylmethoxy]-2-hydroxymethylphenyl}methanol;
7- (4-{[4'-(1-Ethyl-1-hydroxypropyl)-6-methyl-2'-propylbiphenyl-3-ylamino]methyl}-2-hydroxymethylphenyl)methanol;
8- [4-({[4'-(1-Ethyl-1-hydroxypropyl)-6-methyl-2'-propylbiphenyl-3-yl]methylamino}methyl)-2-hydroxymethylphenyl]methanol;
9- [4-({Ethyl-[4'-(1-ethyl-1-hydroxypropyl)-6-methyl-2'-propylbiphenyl-3-yl]amino}methyl)-2-hydroxymethylphenyl]methanol;
10- [4-({[4'-(1-Ethyl-1-hydroxypropyl)-6-methyl-2'-propylbiphenyl-3-yl]propylamino}methyl)-2-hydroxymethylphenyl]-methanol;
11- (2-Hydroxymethyl-4-{2-[6-methyl-2'-propyl-4'-(2,2,2-trifluor-1-hydroxy-1-trifluormethyl-ethyl)biphenyl-3-yl]ethyl}phenyl)-methanol;
12- {2-Hydroxymethyl-4-[6-methyl-2'-propyl-4'-(2,2,2-trifluor-1-hydroxy-1-trifluormethyl-ethyl)biphenyl-3-yloxymethyl]phenyl}-methanol;
13- {2-Hydroxymethyl-4-[6-methyl-2'-propyl-4'-(2,2,2-trifluor-1-hydroxy-1 trifluormethyl-ethyl)biphenyl-3-ylmethoxy]phenyl}-ethanol;
14- (2-Hydroxymethyl-4-{[6-methyl-2'-propyl-4'-(2,2,2-trifluor-1-hydroxy-1-trifluormethyl-ethyl)biphenyl-3-ylamino]methyl}phenyl)methanol;
15- [2-Hydroxymethyl-4-({N-methyl[6-methyl-2'-propyl-4'-(2,2,2-trifluor-1-hydroxy-1-trifluormethyl-ethyl)biphenyl-3-yl]amino}-methyl)phenyl]methanol;
16- [4-({N-Ethyl[6-methyl-2'-propyl-4'-(2,2,2-trifluor-1-hydroxy-1-trifluormethyl-ethyl)biphenyl-3-yl]amino}methyl)-2-hydroxymethylphenyl]methanol;
17- [2-Hydroxymethyl-4-({[6-methyl-2'-propyl-4'-(2,2,2-trifluor-1-hydroxy-1-trifluormethyl-ethyl)biphenyl-3-yl]N-propyl-amino}-methyl)phenyl]methanol;
18- (4-{2-[6-Ethyl-4'-(1-ethyl-1-hydroxypropyl)-2'-propylbiphenyl-3-yl]ethyl}-2-hydroxymethylphenyl)methanol;
19- {4-[6-Ethyl-4'-(1-ethyl-1-hydroxypropyl)-2'-propylbiphenyl-3-ylmethoxy]-2-hydroxymethylphenyl}methanol;
20- (4-{[6-Ethyl-4'-(1-ethyl-1-hydroxypropyl)-2'-propylbiphenyl-3-ylamino]methyl}-2-hydroxymethylphenyl)methanol;
21- [4-({[6-Ethyl-4'-(1-ethyl-1-hydroxypropyl)-2'-propylbiphenyl-3-yl]methylamino}methyl)-2-hydroxymethylphenyl]methanol;
22- [4-({Ethyl-[6-ethyl-4'-(1-ethyl-1-hydroxypropyl)-2'-propylbiphenyl-3-yl]amino}methyl)-2-hydroxymethylphenyl]methanol;
23- [4-({[6-Ethyl-4'-(1-ethyl-1-hydroxypropyl)-2'-propylbiphenyl-3-yl]propylamino}methyl)-2-hydroxymethylphenyl]methanol;
24- (4-{2-[6-Ethyl-2'-propyl-4'-(2,2,2-trifluor-1-hydroxy-1-trifluormethyl-ethyl)biphenyl-3-yl]ethyl}-2-hydroxymethylphenyl)methanol;
25-{4-[6-Ethyl-2'-propyl-4'-(2,2,2-trifluor-1-hydroxy-1-trifluormethyl-ethyl)biphenyl-3-yloxymethyl]-2-hydroxymethylphenyl}-methanol;
26-{4-[6-Ethyl-2'-propyl-4'-(2,2,2-trifluor-1-hydroxy-1-trifluormethyl-ethyl)biphenyl-3-ylmethoxy]-2-hydroxymethylphenyl}-methanol;
27- (4-{[6-Ethyl-2'-propyl-4'-(2,2,2-trifluor-1-hydroxy-1-trifluormethyl-ethyl)biphenyl-3-ylamino]methyl}-2-hydroxymethylphenyl)methanol;
28- [4-({[6-Ethyl-2'-propyl-4'-(2,2,2-trifluor-1-hydroxy-1-trifluormethyl-ethyl)biphenyl-3-yl]methylamino}methyl)-2-hydroxymethylphenyl]methanol;
29- [4-({N-Ethyl[6-ethyl-2'-propyl-4'-(2,2,2-trifluor-1-hydroxy-1-trifluormethyl-ethyl)biphenyl-3-yl] amino}methyl)-2-hydroxymethylphenyl]methanol;
30- [4-({[6-Ethyl-2'-propyl-4'-(2,2,2-trifluor-1-hydroxy-1-trifluormethyl-ethyl)biphenyl-3-yl]-N-propyl-amino}methyl)-2-hydroxymethylphenyl]methanol;
31- (4-{[4'-(1-Ethyl-1-hydroxypropyl)-6,2'-dimethylbiphenyl-3-ylamino]methyl}-2-hydroxymethylphenyl)methanol.

3. Zusammensetzung nach Anspruch 1 oder 2, **dadurch gekennzeichnet, dass** das Vitamin D-Derivat das {4-[6-Ethyl-4'-(1-ethyl-1-hydroxypropyl)-2'-propyl-biphenyl-3-yloxymethyl]-2-hydroxymethyl-phenyl}-methanol ist.

4. Zusammensetzung nach einem der Ansprüche 1 bis 3, **dadurch gekennzeichnet, dass** sie für eine topische Anwendung vorgesehen ist.

5. Zusammensetzung nach einem der Ansprüche 1 bis 4, **dadurch gekennzeichnet, dass** der Wirkstoff in einem Lösemittel solubilisiert ist.

6. Zusammensetzung nach Anspruch 5, **dadurch gekennzeichnet, dass** das Lösemittel unter absolutem Ethanol, Oleyl-macrogol-6-glyceriden, Macrogol- 15-hydroxystearat, hydriertem Ricinusöl PEG 40, PEG 400, Dimethylisosorbid, PPG 15-stearylether, Ethoxydiglycol, N-Methyl-2-pyrrolidon und deren Gemischen ausgewählt ist.

7. Zusammensetzung nach Anspruch 5 oder 6, **dadurch gekennzeichnet, dass** das Lösemittel ein Gemisch aus absolutem Ethanol und mindestens einem Colösemittel ist, das unter Oleyl-macrogol-6-glyceriden, Macrogol-15-hydroxystearat, hydriertem Ricinusöl PEG 40, PEG 400, Dimethylisosorbid, PPG 15-stearylether, Ethoxydiglycol, N-Methyl-2-pyrrolidon und deren Gemischen ausgewählt ist.

8. Zusammensetzung nach einem der Ansprüche 1 bis 7, **dadurch gekennzeichnet, dass** sie ein Additiv zur Verbesserung der Beschaffenheit enthält, das unter Isopropylpalmitat, Isopropylmyristat, C12-15-Alkylbenzoat und deren Gemischen ausgewählt ist.

9. Zusammensetzung nach einem der Ansprüche 1 bis 8, **dadurch gekennzeichnet, dass** der Mengenanteil des Vitamin D-Derivats in solubilisierter Form im Bereich von 0,00001 bis 5 Gew.-%, bezogen auf das Gesamtgewicht der Zusammensetzung, liegt.

10. Zusammensetzung nach einem der Ansprüche 1 bis 9, **dadurch gekennzeichnet, dass** der Mengenanteil des Vitamin D-Derivats in solubilisierter Form im Bereich von 0,0001 bis 3 Gew.-% liegt.

11. Zusammensetzung nach einem der Ansprüche 1 bis 10, **dadurch gekennzeichnet, dass** der Mengenanteil des Vitamin D-Derivats in solubilisierter Form im Bereich von 0,0003 bis 1 Gew.-% liegt.

12. Zusammensetzung nach einem der Ansprüche 5 bis 11, **dadurch gekennzeichnet, dass** der Mengenanteil des Lösungsmittels im Bereich von 1 bis 25 Gew.-%, bezogen auf das Gesamtgewicht der Zusammensetzung, vorzugsweise im Bereich von 1 bis 20 Gew.-% und insbesondere im Bereich von 1 bis 15 Gew.-% liegt.

13. Zusammensetzung nach einem der Ansprüche 1 bis 12, **dadurch gekennzeichnet, dass** sie ferner ein Antioxidationsmittel enthält, das unter Butylhydroxytoluol (BHT), Butylhydroxyanisol (BHA), α-Tocopherol DL und Propylgallat ausgewählt ist.

14. Zusammensetzung nach einem der Ansprüche 1 bis 13, **dadurch gekennzeichnet, dass** sie ein lipophiles reizlinderndes Mittel enthält, das unter dem Acetat von α-Tocopherol DL, Teebaumöl, Extrakt aus grünem Tee und Calendula-Extrakt ausgewählt ist.

15. Verwendung eines elastomeren Polyorganosiloxans und als Wirkstoff mindestens eines Vitamin D-Derivats der allgemeinen Formel (I), wobei der Wirkstoff in solubilisierter Form vorliegt, für die Herstellung einer wasserfreien pharmazeutischen Zusammensetzung nach Anspruch 1, die für die Behandlung von Psoriasis vorgesehen ist.

16. Verwendung nach Anspruch 15, wobei es sich um eine Zusammensetzung handelt, wie sie in einem der Ansprüche 2 bis 14 definiert ist.

17. Verfahren zur Herstellung einer Zusammensetzung nach einem der Ansprüche 1 bis 14, **dadurch gekennzeichnet, dass** es die folgenden Schritte umfasst:
a) Herstellung der Phase A, die das elastomere Organopolysiloxan gegebenenfalls im Gemisch mit einem ergänzenden Siliconöl und/oder einem Additiv zur Verbesserung der Beschaffenheit enthält, bis zur Homogenität;
b) Herstellung der Phase B durch Mischen mindestens eines Vitamin D-Derivats der Formel (I) mit dem Lösemittel bis zur Homogenität,
c) Einbringen der Phase A in die Phase B und anschließend Homogenisierung, bis ein homogenes Gel gebildet ist.

18. Verfahren nach Anspruch 17, **dadurch gekennzeichnet, dass** es sich bei dem Lösemittel um Ethanol handelt.

19. Verfahren nach Anspruch 18, **dadurch gekennzeichnet, dass** es zu Beginn des Schrittes b) einen Schritt umfasst, in dem die verschiedenen Colösemittel mit dem Ethanol vermischt werden, bevor das wirksame Vitamin D-Derivat eingearbeitet wird.

20. Verfahren nach einem der Ansprüche 17 bis 19, **dadurch gekennzeichnet, dass** es in der Kälte durchgeführt wird.
